# EUROPEAN PATENT APPLICATION

(11) **EP 4 129 323 A1**
(43) Date of publication of application: **08.02.2023**
(21) Application number: 21190004.8
(22) Date of filing: 05.08.2021
(51) Int. Cl.: A61K 38/17, C07K 14/74

(54) **A MODIFIED HLA-B57 WITH INCREASED EXPRESSION LEVELS**

(71) Applicant: ImmunOs Therapeutics AG, 8952 Schlieren (CH)
(72) Inventor: MARROQUIN BELAUNZARAN, Osiris, 8592 Schlieren (CH); RENNER, Christoph, 8592 Schlieren (CH); VOGT, Lorenz, 8592 Schlieren (CH)
(74) Representative: Schulz Junghans Patentanwälte PartGmbB

(57) **Abstract**

The invention provides a method of obtaining an HLA-B57 fusion protein, said fusion protein comprising a variant HLA-B57 polypeptide bearing at least, one, or two amino acid substitutions conferring increased stability and expression. The invention also provides an isolated HLA-B57 fusion protein, or a nucleic acid, or a vector encoding said HLA-B57 fusion protein, for use in treating medical conditions such as cancer.

## Description

The present invention relates to modifications to HLA-B57 polypeptides that confers increased expression from mammalian cell lines, for the purpose of producing a medicament.

### Background of the Invention

Classical MHC-I molecules (also known as HLA-I in humans) are dimeric or trimeric structures comprising a membrane-bound heavy chain with three extracellular domains (α1, α2, and α3), bound non-covalently to a β2-microglobulin (β2m) light chain, and optionally, a small peptide associated with the peptide-binding cleft. However, MHC Class I molecules may also disassociate into free heavy chains lacking β2m, referred to as open conformers (Arosa et al. Open conformers: the hidden face of MHC-I molecules, Trends in Immunology 2007 Mar; 28(3):115-23).

The inventors have previously identified the HLA-B57 heavy chain linked to certain immune correlates in HIV infection, as a promising immunomodulatory medicament when delivered in the open conformer format (see WO 2017153438 A1). To obtain isolated HLA-B57 open conformer, the HLA heavy chain must first be co-expressed with β2m. Once purified from the supernatant, the HLA heavy chain / β2m complex may be separated, and the isolated HLA chains purified and subjected to refolding to form open conformers. However, while open conformer HLA-B57 has desirable immunomodulatory qualities, upscaling of manufacturing to industrial quantities has revealed challenges. HLA-B57 heavy chains form oligomers and aggregates in solution, leading to reduced cell viability, and low yields of both the HLA heavy chain / β2m complex, and the derived open conformer.

Based on the above-mentioned state of the art, the objective of the present invention is to provide a pharmaceutically active HLA-B57 heavy chain amenable to high yield recombinant protein expression, and characterized by desirable immunomodulatory properties. This objective is attained by the subject-matter of the independent claims of the present specification, with further advantageous embodiments described in the dependent claims, examples, figures and general description of this specification.

### Summary of the Invention

A first aspect of the invention relates to a method to obtain an HLA-B57 fusion protein comprising a variant of the extracellular domain of an HLA-B57 heavy chain, characterized by favorable manufacturing and immunomodulatory qualities. The method comprises introducing at least one, or two amino acid substitutions into the extracellular domain of a naturally occurring HLA-B57 heavy chain polypeptide. The resulting variant HLA-B57 polypeptide is joined to a stabilizing polypeptide, to obtain a modified, variant HLA-B57 fusion protein which is highly expressed in cell culture. The key amino acid residues identified in the naturally occurring HLA-B57 polypeptide sequence by the invention, are the exchange of an alanine (A), for a glutamate (E) at position 46, and/or a valine (V) or tryptophan (W) for an arginine (R) at position 97. The positions numbers for this, and any other HLA-B57 heavy chain polypeptides referred to herein, are defined sequentially by assigning the glycine (G), serine (S), histidine (H) motif initiating the extracellular domain of the naturally occurring full-length HLA-B57 heavy-chain to the positions 1, 2 and 3, respectively.

Another aspect of the invention is an isolated HLA fusion protein, comprising a variant HLA-B57 polypeptide based on the extracellular domain of a naturally occurring HLA-B57 amino acid sequence as above, but unlike the naturally occurring sequence, characterized by an E at position 46, and an R at position 97, and linked to an IgG Fc portion.

Further aspects of the invention relate to a pharmaceutical composition comprising the isolated HLA fusion protein as specified above, or a nucleic acid, or nucleic acid expression vector encoding said HLA fusion protein, for use as a medicament for the purpose of treating a malignant neoplastic disease. Pharmaceutical compositions according to the invention, comprise at least one of the compounds of the present invention and at least one pharmaceutically acceptable carrier, diluent or excipient.

### Detailed Description of the Invention

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of an RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

The term *nucleic acid expression vector* in the context of the present specification relates to a plasmid, a viral genome, or an RNA, which is used to transfect (in case of a plasmid or an RNA) or transduce (in case of a viral genome) a target cell with a certain gene of interest, or - in the case of an RNA construct being transfected- to translate the corresponding protein of interest from a transfected mRNA. For vectors operating on the level of transcription and subsequent translation, the gene of interest is under control of a promoter sequence and the promoter sequence is operational inside the target cell, thus, the gene of interest is transcribed either constitutively or in response to a stimulus or dependent on the cell's status. In certain embodiments, the viral genome is packaged into a capsid to become a viral vector, which is able to transduce the target cell.

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

Reference to identical sequences without specification of a percentage value implies 100% identical sequences (i.e. the same sequence).

As used herein, the term *pharmaceutical composition* refers to an HLA fusion protein according to the invention, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

In the context of the present specification, the term *peptide linker,* or *amino acid linker* refers to a polypeptide of variable length that is used to connect two polypeptides in order to generate a single chain polypeptide. Exemplary embodiments of linkers useful for practicing the invention specified herein are oligopeptide chains consisting of 1, 2, 3, 4, 5, 10, 20, 30, 40 or 50 amino acids. A non-limiting example of an amino acid linker is the flexible, glycine-rich peptide GGGGSGGGGS (SEQ ID NO 003) used to link a variant HLA-B57 polypeptide with a stabilizing peptide to provide an HLA fusion protein in the examples.

The term *human leukocyte antigen, HLA, HLA heavy chain,* or *HLA alpha chain* in the context of the present specification relates to the family of proteins encoded by the *Class I Major histocompatibility (MHC) antigen* gene family on chromosome 6. The HLA heavy chain can be a monomer, or form a part of dimeric structures comprising a heavy chain with three extracellular domains (α1, α2, and α3), bound non-covalently to a β2m light chain, or optionally, trimeric structures wherein a small peptide epitope is associated at the peptide-binding cleft.

The term or *naturally occurring HLA-B57 (HLA-B*57, B57, HLA-B57, HLA-B57 polypeptide)* refers specifically to the products of the MHC Class I HLA-B57 subfamily of genes, encoding numerous similar proteins. The HLA-B57 family currently encompasses 221 known alleles with unique nucleic acid sequences, and several dozen unique protein sequences, numbering HLA-B*57:01 to HLA-B*57:141 (the sequence for which are known, and may be retrieved, for example, by entering the search term "B*57" into the MGT/HLA Allele Query Form provided by the European Bioinformatics Institute Immuno Polymorphism Database, Robinson J. et al. 2013 Nucleic Acids Res. 41:D1234, https://www.ebi.ac.uk/ipd/imgt/hla/allele.html). Full length *naturally occurring HLA-B57* comprise an extracellular domain comprising an α1, an α2, and an α3 domain, a connecting peptide region, a transmembrane domain, and an intracellular domain.

The term *extracellular domain* as applied to an HLA-B57 polypeptide, or variant HLA-B57 polypeptide in the context of the specification refers to the extracellular portion of the HLA-B57 heavy chain protein. It comprises an α1, α2, and an α3 domain, regions of which are essential for receptor ligand interactions which mediate the immunomodulatory effects of an HLA fusion protein according to the invention. The *extracellular domain* excludes the transmembrane domain, and the intracellular domain, though it may optionally further comprise:
- a secretory signal, for example, the initial 24 amino acids present in most naturally occurring HLA-B57 polypeptides annotated as a signal peptide, or an alternative secretion signal designed for efficient HLA fusion protein secretion, such as SEQ ID NO 019, and/or
- C-terminal to the alpha 1, alpha 2 and alpha 3 domains of the extracellular domain of natural HLA-B57 protein sequence, the initial 6 residues of the HLA-B57 "connecting peptide" region joining the alpha 3 domain, with the transmembrane domain, and present in most naturally occurring full-length variants of the HLA-B57 polypeptide.

The term *variant HLA-B57 polypeptide* refers to a variant polypeptide obtained by introducing amino acid modifications into the extracellular domain of a natural *HLA-B57* polypeptide sequence as specified above. Said *variant HLA-B57 polypeptide* in the terminology used in the current specification consists of, or comprises a variant of the portion of the extracellular domain of a natural HLA-B57 polypeptide sequence required for interaction with HLA-B57 ligands, particularly comprising at least the alpha 1, alpha 2 and alpha 3 domains of a naturally occurring HLA-B57 protein sequence.

Said *variant HLA-B57 polypeptide* is further characterized by:
- a glutamate (E) at amino acid residue position 46, and
- an arginine (R) at amino acid residue position 97.

The amino acid numbering for HLA-B57 used herein is defined sequentially by assigning the initial G,S,H motif of an extracellular domain of the variant, or naturally occurring HLA-B57 heavy-chain with the sequence SEQ ID NO 001 to the positions 1, 2 and 3, respectively.

Said *variant HLA-B57 polypeptide* optionally further comprises:
- a secretory signal, for example, the initial 24 amino acids present in most naturally occurring HLA-B57 polypeptides annotated as a signal peptide, or an alternative secretion signal designed for efficient HLA fusion protein secretion, such as SEQ ID NO 019, and/or
- C-terminal to the alpha 1, alpha 2 and alpha 3 domains of the variant extracellular domain of a naturally occurring HLA-B57 protein sequence, the initial 6 residues of the HLA-B57 "connecting peptide" region joining the alpha 3 domain, with the transmembrane domain, and present in naturally occurring, full-length HLA-B57 polypeptides.

Said *variant HLA-B57 polypeptide* lacks, or does not encode:
- the transmembrane domain, and
- the intracellular domain of a naturally occurring HLA-B57 protein sequence.

Said *variant HLA-B57 polypeptide* preferably lacks, or does not encode:
- the last 3 to 11 residues, particularly the last 5 residues of the "connecting peptide" region of the extracellular domain present in most full length naturally HLA-B57 protein sequences joining the alpha 3 domain, and the transmembrane domain.

In the context of the present specification, the terms *HLA fusion protein* or *HLA-B57 fusion protein* refer to a polypeptide which comprises, or essentially consists of a variant HLA-B57 extracellular domain polypeptide, joined to a stabilizing Fc polypeptide domain, optionally by means of a peptide linker. An *HLA fusion protein* according to the invention, comprising the variant HLA-B57 polypeptide as specified in the 5 preceding paragraphs, is sometimes referred to in the examples herein as *HLA-B57(^{A46E, V97R}).* The term encompasses both an *HLA fusion protein* in complex with a *β2-microglobulin* polypeptide secreted from a cell culture, and the purified, open conformer *HLA fusion protein* which is has been separated from β2-microglobulin. The term *HLA fusion protein* encompasses both a monomer form, comprising a single HLA-B57 polypeptide joined to a stabilizing immunoglobulin (Ig) Fc domain, or a dimer formed by association of a first HLA fusion protein monomer, and a second HLA fusion protein monomer, for example, via association of Ig Fc domains.

In the context of the present specification, the term *β2-microglobulin protein (β2m protein, β2m, B2m, HLA light chain)* refers to the beta (β) chain, also known as the light chain, of the MHC class I heterodimer. The term *β2-microglobulin protein* encompasses firstly, a pre-processing *β2-microglobulin protein* comprising a secretory signal, for example, the sequence of Uniprot P61769, or the sequence SEQ ID NO 014, and secondly, the post-secretion form of the protein, in which a secretory signal portion of the protein has been removed by cleavage as the polypeptides are exported from the cell.

In the context of the present specification, the term *open conformer* (OC) refers to an HLA heavy chain molecule, particularly an HLA fusion protein according to the invention, lacking association with a β2m molecule. To obtain an HLA fusion protein *open conformer* by means of the recombinant protein manufacturing processes used herein, a molecule comprising an HLA heavy chain polypeptide is expressed together with a β2m protein in a mammalian cell, and the resulting HLA : β2m complex is then separated under acidic conditions, after which the β2m-free HLA fusion protein is purified by size exclusion. In the context of the present specification, the terms *HLA-B57.OC* or *HLA-B57*^{*(A46E*/}*^{V97R)}.OC* refer to HLA fusion proteins comprising the non-variant, and variant HLA heavy chain polypeptide linked to an IgG Fc polypeptide respectively (in which ^{*(A46E*/*V97R)*} denotes the presence of the variant HLA polypeptide according to the invention), which has been separated from the β2m protein.

In the context of the present specification, the term *secretory signal, secretory signal peptide* or *signal sequence* refers to an N-terminal leader sequence initiating the open reading frame (ORF) of a polypeptide, usually about 6-30 amino acids in length. In rare cases, a *secretory signal* is placed at the C-terminus of a polypeptide. *Secretory signals* are sometime referred to as targeting signals, localization signals, transit peptides, leader sequences, or leader peptides. *Secretory signals* which enable efficient secretion of a polypeptide from a cell are well known in the art, and may be included in the ORF of a recombinant protein in order to facilitate export of a polypeptide to the supernatant in cell-based polypeptide manufacturing system, allowing purification of a polypeptide from the cell supernatant. Upon translation of the mRNA encoding the *secretory signal,* it is recognized by a cytosolic protein mediating transfer of the mRNA-ribosome complex to a channel protein in the endoplasmic reticulum (ER). The newly synthesized polypeptide comprising the *secretory signal peptide* is translocated to the ER lumen through the channel protein, entering the cell secretion pathway. *Signal sequences* of particular use according to the invention are those that are cleaved from the final polypeptide product following translation, such as those used in the examples such as SEQ ID NO 019, or that comprised within SEQ ID NO 020.

In the context of the present specification, the term *immunoglobulin crystallizable fragment (Fc) region,* or *Ig Fc* refers to a fraction of an antibody, or immunoglobulin (Ig), comprised of a C_{H}2 and a C_{H}3 domain. *Ig Fc* encompasses both a monomer, or a dimer comprising two *Ig Fc,* covalently linked by disulfide bonds. In the context of the HLA fusion protein according to the invention, disulfide bonds can join two HLA fusion proteins molecules, each comprising *Ig Fc* domains. The presence of the *Ig Fc* in the HLA fusion protein facilitates increased solubility, stability, avidity, half-life, and from a technological point of view, cost-effective production and purification in mammalian systems (protein A or G purification).

In the context of the present specification, the term *checkpoint inhibitory agent* encompasses a cancer immunotherapy agent capable of disrupting an inhibitory signalling cascade that limits immune cell activation, and in particular T cell activation, known in the art as an immune checkpoint mechanism, particularly a *checkpoint inhibitor antibody* or an antibody-like molecule, such as an antibody fragment, a diabody, or a single variable chain antibody fragment. Examples of *checkpoint inhibitory agents* include, for example, an antibody, antibody like molecule, or natural ligand receptor which binds specifically to CTLA-4 (Uniprot P16410), PD-1 (Uniprot Q15116), PD-L1 (Uniprot Q9NZQ7), B7H3 (CD276; Uniprot Q5ZPR3), VISTA (Uniprot Q9H7M9), TIGIT (UniprotQ495A1), or TIM-3 (HAVCR2, Uniprot Q8TDQ0), CD137 (41BB, Uniprot Q07011), CD40 (Uniprot Q09LL4), CD27 (Uniprot P26841), OX40 (CD134, UniprotP43489), NKG2A (Uniprot P26715), CD86 (Uniprot P42081), CD80 (Uniprot P33681), or LAG-3 (Uniprot P18627).

### Improved methods for producing an HLA-B57 fusion protein

A first aspect of the invention relates to **a method for producing an HLA-B57 fusion protein** with improved mammalian cell system expression properties, said HLA-B57 fusion protein comprising a variant HLA-B57 polypeptide, joined to a stabilizing polypeptide. The method involves altering specific amino acids in the extracellular domain of a naturally occurring HLA-B57 heavy-chain polypeptide, to provide a variant which retains desirable immunomodulatory qualities, but is easier to manufacture as a result of enhanced stability, less propensity to form aggregates in solution, and higher expression titers.

In some embodiments, the method according to this aspect of the invention comprises introducing into a cell, a nucleic acid sequence encoding a polypeptide comprising a variant of the extracellular domain of a naturally occurring HLA-B57 heavy-chain, fused to a stabilizing polypeptide, wherein the **variant HLA-B57 heavy chain polypeptide** (but not the **naturally occurring** HLA-B57 heavy-chain polypeptide it is derived from) is characterized by a **glutamate (E) at position 46.** The amino acid position numbering according to the invention is defined sequentially by assigning the initial glycine (G), serine (S), histidine (H) motif of the extracellular domain of a naturally occurring HLA-B57 heavy-chain to positions 1, 2 and 3, respectively. In other words, the secretory signal peptide is not included in the numbering.

In some embodiments, the method according to this aspect of the invention, comprises introducing into a cell a nucleic acid sequence encoding an HLA fusion protein comprising a variant of the extracellular domain of a naturally occurring HLA-B57 heavy-chain fused to a stabilizing polypeptide. According to this embodiment, the **variant HLA-B57 heavy chain polypeptide** (but not the **naturally occurring** HLA-B57 heavy-chain polypeptide it is derived from) is characterized by **an arginine (R) at position 97.**

In certain embodiments of the method according to the invention, the nucleic acid sequence encoding the HLA fusion protein encodes a variant HLA-B57 polypeptide which is identical to the extracellular portion of any naturally occurring HLA-B57 heavy chain polypeptide which:
- comprises a full-length extracellular domain, and
- is not characterized by an E at position 46, and /or an R at position 97.

Another aspect of the invention is a method for producing an HLA fusion protein comprising as a first step, introducing amino acid substitutions into the extracellular domain of any naturally occurring, full length HLA-B57 heavy-chain polypeptide, to provide a variant HLA-B57 polypeptide characterized by an E at position 46, and an R at position 97. Depending on the HLA-B57 sequence, this may entail introducing an amino acid substitution encoding an E at position 46 (A46E), and/or introducing an amino acid substitution encoding an R at position 97 (V97R or W97R). The method according to this aspect of the invention comprises a second step, introducing into a cell, a nucleic acid sequence encoding an HLA fusion protein comprising said variant HLA-B57 polypeptide joined to a stabilizing polypeptide.

The method to produce an HLA fusion protein according to the first, or the second aspect of the invention, comprises introducing into the same cell, a nucleic acid encoding a β2m polypeptide sequence, to increase the expression level, and secretion of the HLA fusion protein in the form an HLA heavy-chain / β2m complex, also referred to as HLA.β2m. HLA.β2m can be further dissociated to provide isolated HLA open conformer molecules lacking β2m, also referred to as HLA-B57.OC. The nucleic acids encoding the HLA fusion protein, and the β2m polypeptide, according to these aspects of the invention, are under control of a promoter sequence operable in said cell. The cell is then cultured under conditions where the HLA-B57 and the β2m encoding nucleic acid sequences are expressed, to provide the HLA.β2m complex.

In particular embodiments of the first or second aspect of the invention, the stabilizing polypeptide joined to the variant HLA-B57 polypeptide, is an Ig Fc polypeptide. In more particular embodiments, the stabilizing peptide is an IgG Fc polypeptide. In still more particular embodiments, the stabilizing peptide is an IgG4 Fc polypeptide. In even more particular embodiments, the stabilizing peptide is an IgG4 Fc polypeptide with the sequence SEQ ID NO 004. In other embodiments which the inventors consider feasible, the stabilizing polypeptide is an albumin polypeptide. In other embodiments considered feasible by the inventors, the stabilizing polypeptide is a PEG-containing compound.

In particular embodiments of the methods to produce an HLA heavy chain fusion protein according to the invention, the obtained HLA fusion protein comprises, from the N' to the C' terminus of the fusion protein:
i. a variant HLA-B57 polypeptide,
ii. a peptide linker between 5 and 20 amino acids in length, more particularly a peptide linker with the sequence SEQ ID NO 003, and
iii. a stabilizing polypeptide.

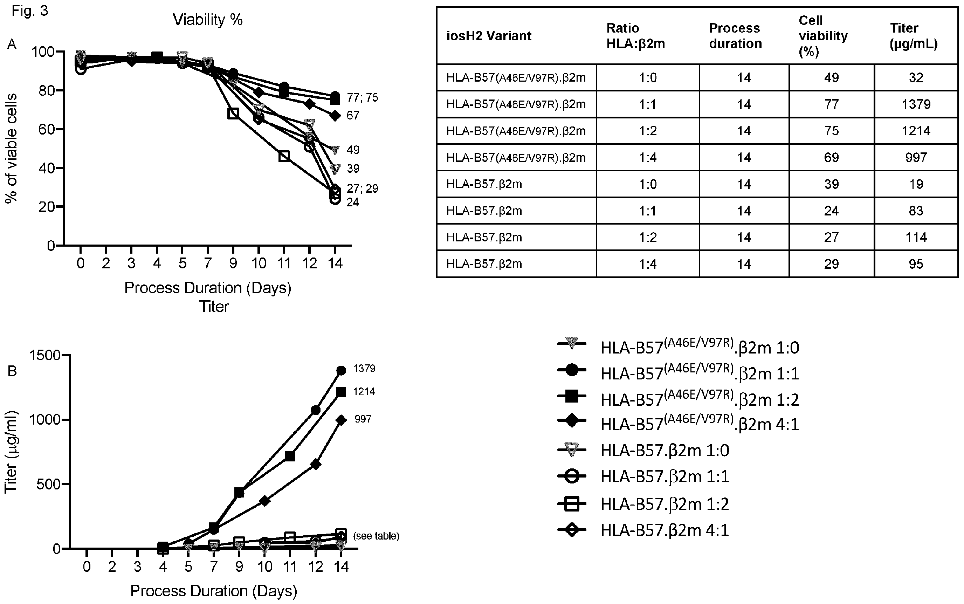

In certain embodiments of the methods to produce an HLA heavy chain fusion protein according to the invention, the HLA fusion protein nucleic acid sequence, and the β2m nucleic acid sequence, are present on a single nucleic acid vector molecule (for example, on a single plasmid). In particular embodiments, the HLA fusion protein nucleic acid sequence, and the β2m nucleic acid sequence are present on different nucleic acid vector molecules (for example, expressed from two different plasmids). In such embodiments, the optimal ratio of the nucleic acid vector comprising the HLA fusion protein nucleic acid sequence with respect to the nucleic acid vector comprising the β2m nucleic acid sequence is ≥ 1 and ≤ 2, particularly the ratio is > 1 and < 2, as demonstrated in Fig. 3 of the examples.

In some embodiments of the methods to produce an HLA fusion protein according to the invention as specified above, the method comprises the following additional steps:
a. a purification step, wherein an HLA fusion protein associated with β2m protein, in other words, an HLA fusion protein / β2m protein complex, is purified from the supernatant of the cell; and/or
b. a dissociation step, wherein the purified HLA fusion protein / β2m protein complex is dissociated under conditions such that an isolated HLA fusion protein separates from an isolated β2m protein.

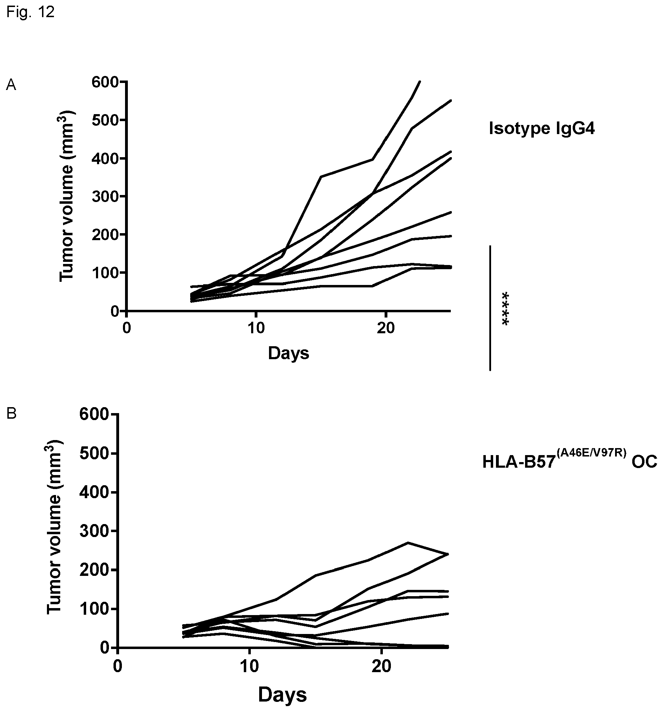

Step a. will provide an HLA fusion protein associated with β2m protein, and step b. an HLA fusion protein disassociated from β2m protein - the open conformer demonstrated to antagonize tumor growth when administered *in vivo* in Fig. 12. The data provided in the examples demonstrate an example of the purification step according to this aspect of the invention, wherein Protein G Sepharose is used to capture the HLA fusion protein comprising an IgG4 polypeptide from cell supernatants, following a first elution with a pH 2.8 IgG elution buffer to isolate a variant HLA-B57 fusion protein associated to β2m protein (also referred to as HLA:β2m or HLA fusion protein : β2m protein complex).

In some embodiments of the methods for producing an isolated HLA fusion protein according to either the first or second aspect of the invention, a further dissociation step is carried out to provide an open conformer. This may be achieved, for example, by exposure of HLA fusion protein : β2m protein complex to acidic conditions, particularly approximately pH 3, allowing separation of open conformer HLA fusion protein from the β2m protein by size exclusion chromatography. In some embodiments of the methods, the method may further comprise a desalting step, wherein following dissociation of β2m from the HLA fusion protein / β2m protein complex, the purified HLA fusion proteins are brought to a physiological pH, allowing correct folding of the protein.

In certain embodiments of the methods to produce an HLA fusion protein according to either the first or second aspect of the invention, the naturally occurring extracellular domain of an HLA-B57 heavy-chain polypeptide forming the base sequence for the variant HLA-B57 polypeptide, is characterized by an A at position 46. This embodiment encompasses the use of any HLA-B57 heavy chain as a starting point for creating the variant HLA heavy chain polypeptide, except those where position 46 is already E (for example, HLA-B57:16, HLA-B57:45, HLA-B57:51, or HLA-B57:69). In other words, in order to obtain the variant sequence from the naturally occurring sequence, at least one (A46E), and possibly two amino acid substitutions (both A46E and either V97R, or W97R) are introduced at the indicated sites of the naturally-occurring HLA-B57 extracellular domain.

In alternative embodiments of the methods to produce an HLA fusion protein according to either the first or second aspect of the invention, the extracellular domain of the naturally occurring HLA-B57 heavy-chain is characterized by a V at position 97. This encompasses any HLA-B57 heavy chain except those where position 97 is already R (for example, HLA-B57:05, HLA-B57:82, HLA-B57:83, HLA-B57:118, or HLA-B57:131), or where position 97 is a W (HLA-B57:11). In these embodiments, in order to obtain the variant sequence from the naturally occurring sequence, at least one (V97R), and possibly two amino acid substitutions (both V97R and A46E) are introduced at the indicated sites.

In other embodiments of the methods to produce an HLA fusion protein according to either the first or second aspect of the invention, the extracellular domain of the naturally occurring HLA-B57 heavy-chain is characterized by an A at position 46, and either a V, or a W at position 97. In other words, the naturally occurring HLA-B57 haplotype may be any HLA-B57 heavy chain except those where position 46 is already an E (for example, HLA-B57:16, HLA-B57:45, HLA-B57:51, HLA-B57:69) or position 97 is already an R (for example, HLA-B57:05, HLA- HLA-B57:82, HLA-B57:83, HLA-B57:118, or HLA-B57:131). In these embodiments, in order to obtain the variant sequence from the naturally occurring sequence, two amino acid substitutions, an A46E and either a V97R or W97R, are introduced at the indicated sites to provide the variant sequence.

In particular embodiments of the methods to produce an HLA fusion protein according to the invention, two amino acid substitutions A46E, and V97R are introduced into a naturally occurring polypeptide sequence of any HLA-B57 extracellular domain which is characterized by both an A at position 46, and a V at position 97. This embodiment encompasses the use of any HLA-B57 haplotype as a starting point for obtaining the variant HLA polypeptide domain of the fusion protein, except those where position 46 is already an E (for example, HLA-B57:16, HLA-B57:45, HLA-B57:51, or HLA-B57:69), or where position 97 is already R (HLA-B57:05, HLA-B57:82, HLA-B57:83, HLA-B57:118, HLA-B57:131), or where position 97 is a W (HLA-B57:11).

In more particular embodiments of the methods to produce an HLA fusion protein according to either the first or second aspect of the invention, the variant HLA-B57 polypeptide portion of the HLA fusion protein comprises the sequence SEQ ID NO 002. In other words, the method to produce an HLA fusion protein according to this embodiment involves introducing into a cell a nucleic acid encoding the β2m protein, and a nucleic acid encoding an HLA fusion protein (comprising a variant HLA heavy chain polypeptide joined to an IgG4 Fc molecule), where the variant HLA-B57 polypeptide is identical to the naturally occurring HLA-B57:01 sequence SEQ ID NO 001, apart from amino acid substitutions that encode an E at position 46, and an R at position 97. This cell is then cultured in conditions amenable to expression of both proteins, to provide an HLA fusion protein / β2m protein complex.

In particular embodiments of the methods to obtain an HLA fusion protein according to either the first or second aspect of the invention, the nucleic acid sequence encoding an HLA fusion protein, encodes a polypeptide sequence comprising the sequence SEQ ID NO 015, the fusion protein as it is found following secretion from mammalian cells, lacking a secretion signal. In more particular embodiments of the methods to obtain an HLA fusion protein, the nucleic acid sequence encodes an HLA fusion protein polypeptide sequence which comprises the sequence SEQ ID NO 005, or SEQ ID NO 020, where the HLA fusion polypeptide sequence further comprises one of two secretory signals the inventors found to facilitate efficient secretion of the HLA fusion protein from the cell. In still more particular embodiments of the method, the nucleic acid encodes an HLA polypeptide which essentially consists of SEQ ID NO 005.

In some embodiments of the methods to obtain an HLA fusion protein, the nucleic acid sequence encoding an HLA fusion protein comprises the sequence SEQ ID NO 016, preferably associated with an additional nucleic acid encoding a secretion signal sequence.

In further particular embodiments of the methods to produce an HLA heavy chain fusion protein, the HLA fusion protein nucleic acid sequence encodes a secretory signal. In still more particular embodiments, it encodes a secretory signal 16 to 30 amino acids in length, positioned N-terminal to the encoded variant HLA-B57 polypeptide sequence. In still more particular embodiments, the secretory signal encoded by HLA fusion protein nucleic acid sequence has the sequence SEQ ID NO 019.

In particular embodiments of the methods to obtain an HLA fusion protein, the nucleic acid sequence encoding an HLA fusion protein comprises the sequence SEQ ID NO 006, which encodes a useful secretion signal preceding the HLA fusion protein. In more particular embodiments of the methods to obtain an HLA fusion protein, the nucleic acid sequence encoding an HLA fusion protein essentially consists of the sequence SEQ ID NO 006. The secretory signal encoded by the HLA fusion protein nucleic acid sequence is removed by cleavage during the process of secretion from the cell.

In some embodiments of any one of the aspects of the method to obtain an HLA fusion protein specified above, the cell producing the HLA fusion protein is a eukaryotic cell. In particular embodiments, the cell is a mammalian cell. In more particular embodiments of the method to produce an HLA fusion protein as demonstrated in the examples, a Chinese hamster ovarian (CHO) cell is used to express the recombinant fusion protein product, though the inventors predict other cell types such as insect cells, or particularly other mammalian cell types, can feasibly produce an HLA fusion protein according to the invention.

### Improved high yield HLA-B57 fusion protein

A next aspect of the invention is an **isolated HLA fusion protein** comprising firstly, a **variant HLA-B57 polypeptide** which differs by at least 1, or 2 amino acids from the extracellular domain (as specified according to the definition of the term *variant HLA-B57 polypeptide)* of a naturally occurring HLA-B57 heavy-chain polypeptide. Said **variant HLA-B57 polypeptide is characterized by an E at position 46, and an R at position 97,** wherein the amino acids residues are numbered from the G,S,H motif located at the N-terminal region of extracellular alpha 1 domain (using amino acid numbering as specified in the definition of the term *variant HLA-B57 polypeptide).* Secondly, the isolated HLA fusion protein comprises a **stabilizing polypeptide**, for example, an Ig Fc. In particular embodiments, the stabilizing polypeptide is an IgG Fc. In even more particular embodiments, the stabilizing peptide is an **IgG4 Fc**. In even more particular embodiments, the stabilizing peptide is an **IgG4 Fc** with desirable pharmaceutical qualities, with the polypeptide sequence designated SEQ ID NO 004.

In some embodiments of isolated HLA fusion protein according to the invention, it comprises a variant HLA-B57 polypeptide obtained by introducing amino acid substitutions according to the method according to any one of the aspects or embodiments specified in the section *Improved methods for production* of *an HLA-B57 fusion protein,* into the extracellular portion of a naturally occurring HLA-B57 polypeptide, such that it is characterized by an E at position 46 and an R at position 97.

In particular embodiments, the isolated HLA fusion protein of the invention comprises a variant HLA-B57 polypeptide comprising the sequence SEQ ID NO 002. In more particular embodiments, the isolated HLA fusion protein of the invention comprises a variant HLA-B57 polypeptide essentially consisting of the sequence designated SEQ ID NO 002.

In further particular embodiments of the isolated HLA-B57 fusion protein according to the invention, it essentially consists of a variant HLA-B57 polypeptide and a stabilizing polypeptide joined by a peptide linker. In particular embodiments, the peptide linker is between 5 and 20 amino acids in length. In more particular embodiments this joining peptide linker has the sequence SEQ ID NO 003.

In still further particular embodiments, the isolated HLA fusion protein comprises the sequence designated SEQ ID NO 015. In still more particular embodiments, the isolated HLA fusion protein essentially consists of a polypeptide with the sequence designated SEQ ID NO 015.

### Variant HLA-B57 polypeptides

All naturally occurring HLA-B57 heavy chains comprising a full length extracellular domain, are characterized by either an alanine (A) at position 46, and/or a valine (V) or tryptophan (W) at position 97. The sequences of the HLA-B57 family of proteins are retrievable by entering the search term "B*57" into the Alleles Resource of the Immuno polymorphism database (IBD, https://www.ebi.ac.uk/ipd/imgt/hla/allele.html, Robinson J. et al. 2013 Nucleic Acids Res. 41:D1234). A majority are characterized by an A at position 46, and a V or W at position 97. Therefore, the method according to the first and second aspect of the invention may comprise introducing either one, or two amino acid substitutions into the original protein sequence to provide a variant HLA-B57 polypeptide. In the figures presented in the examples, the manufacture of such an HLA fusion protein according to the invention, first as a complex of a variant HLA-B57 polypeptide fused to an IgG4 Fc in complex with β2m protein, and as an open conformer variant HLA-B57 polypeptide IgG4 Fc fusion protein disassociated from β2m protein, is improved by amino acid substitutions at position E46 and R97, when compared to an otherwise identical structures with an unmodified HLA-B*57:01 sequence.

Certain domains of the naturally occurring HLA-B57 heavy chain not required for cognate ligand interactions, the intracellular domain, and the transmembrane domain, are absent from the HLA fusion protein according to any one of the aspects of the invention herein. In particular embodiments of the method to produce an HLA fusion protein, or the isolated HLA fusion protein according to the invention, the variant HLA-B57 polypeptide comprises the core structure of the extracellular portion of the naturally occurring HLA heavy chain protein sequence, comprising the alpha 1, 2, and 3 domains, as this portion confers the fusion protein with the ability to interact with surface molecules on target cells.

In particular embodiments of the methods to produce an HLA fusion protein, or the isolated HLA fusion protein according to the invention, the variant HLA-B57 polypeptide comprised in the HLA fusion protein includes the alpha 1, 2 and 3 domains of the naturally occurring HLA heavy chain protein. In more particular embodiments of the method to produce an HLA fusion protein, or the isolated HLA fusion protein according to the invention, the variant HLA-B57 polypeptide protein is the alpha 1, 2 and 3 domains of the naturally occurring HLA heavy chain protein, excepting the C-terminal isoleucine-valine dipeptide, preceded by the threonine-valine-proline residues of the extracellular domain, within the HLA-B57 region preceding the transmembrane domain sometimes annotated, or referred to, as the "connecting peptide".

Structural data suggests that HLA-B57 interacts with ligands such as Killer immunoglobulin-like receptors (KIR) and leukocyte immunoglobulin-like receptors (LILR) via regions distant from the transmembrane region. Amino acids close to the membrane do not generally interact with receptors. Furthermore, the inventors surmise that the high content of hydrophobic amino acids within the 5 C-terminal amino of the extracellular domain of naturally occurring HLA heavy chain sequences specified in the preceding paragraph, is likely to introduce undesirable properties into recombinant fusion proteins, such as a tendency towards protein aggregation, which can then affect the production, purification, stability and toxicity in downstream production processes.

In particular embodiments of the methods to produce an HLA fusion protein, or the isolated HLA fusion protein according to the invention, the variant HLA-B57 heavy chain polypeptide is derived from an extracellular domain polypeptide of any HLA-B57 heavy chain from HLA-B*57:01 to HLA-B*57:141 (with the exception of truncated haplotypes lacking the full extracellular domain sequence). Amino acid substitutions in the naturally occurring polypeptide, resulting in position 46 (counting from the G,S,H motif) of the extracellular domain as an E, and the amino acid at position 97 as an R, provide a variant HLA heavy chain polypeptide, or fusion protein according to the invention. In addition to SEQ ID NO 001, suitable naturally occurring HLA-B57 heavy chain protein sequences comprising the required alpha 1, 2, and 3 domain of the extracellular portion of the polypeptide as specified in the definition of the term *variant HLA-B57 polypeptide,* that may serve as a starting point for constructing the variant HLA-B57polypeptide, or HLA fusion protein according to the invention can be found in public sequence repositories. These include sequences selected from, but not limited to, the HLA-B57 heavy chains in the first column of the following table, which may be retrieved from the EMBL-EBI laboratory's IPD database (as cited in the definition of the term *HLA-B57* in the *Terms and Definitions* section):

| Allele | Source |
|---|---|
| B57:02:01 | https://www.ebi.ac.uk/cgi-bin/ipd/imgt/hla/get_allele.cgi?B*57:02:01:01 |
| B57:03:01 | https://www.ebi.ac.uk/cgi-bin/ipd/imgt/hla/get_allele.cgi?B*57:03:01:01 |
| B57:04:01 | https://www.ebi.ac.uk/cgi-bin/ipd/imgt/hla/get_allele.cgi?B*57:04:01 |
| B57:05 | https://www.ebi.ac.uk/cgi-bin/ipd/imgt/hla/get_allele.cgi?B*57:05 |
| B57:06 | https://www.ebi.ac.uk/cgi-bin/ipd/imgt/hla/get_allele.cgi?B*57:06 |
| B57:08 | https://www.ebi.ac.uk/cgi-bin/ipd/imgt/hla/get_allele.cgi?B*57:08 |
| B57:09 | https://www.ebi.ac.uk/cgi-bin/ipd/imgt/hla/get_allele.cgi?B*57:09 |
| B57:11 | https://www.ebi.ac.uk/cgi-bin/ipd/imgt/hla/get_allele.cgi?B*57:11 |
| B57:12 | https://www.ebi.ac.uk/cgi-bin/ipd/imgt/hla/get_allele.cgi?B*57:12 |
| B57:15 | https://www.ebi.ac.uk/cgi-bin/ipd/imgt/hla/get_allele.cgi?B*57:15 |
| B57:29 | https://www.ebi.ac.uk/cgi-bin/ipd/imgt/hla/get_allele.cgi?B*57:29 |
| B57:78 | https://www.ebi.ac.uk/cgi-bin/ipd/imgt/hla/get_allele.cgi?B*57:78 |
| B57:82 | https://www.ebi.ac.uk/cgi-bin/ipd/imgt/hla/get_allele.cgi?B*57:82 |
| B57:83 | https://www.ebi.ac.uk/cgi-bin/ipd/imgt/hla/get_allele.cgi?B*57:83 |
| B57:84 | https://www.ebi.ac.uk/cgi-bin/ipd/imgt/hla/get_allele.cgi?B*57:84 |
| B57:86 | https://www.ebi.ac.uk/cgi-bin/ipd/imgt/hla/get_allele.cgi?B*57:86 |
| B57:91 | https://www.ebi.ac.uk/cgi-bin/ipd/imgt/hla/get_allele.cgi?B*57:91 |
| B57:92 | https://www.ebi.ac.uk/cgi-bin/ipd/imgt/hla/get_allele.cgi?B*57:92 |
| B57:93 | https://www.ebi.ac.uk/cgi-bin/ipd/imgt/hla/get_allele.cgi?B*57:93 |
| B57:94 | https://www.ebi.ac.uk/cgi-bin/ipd/imgt/hla/get_allele.cgi?B*57:94 |
| B57:95 | https://www.ebi.ac.uk/cgi-bin/ipd/imgt/hla/get_allele.cgi?B*57:95 |
| B57:96 | https://www.ebi.ac.uk/cgi-bin/ipd/imgt/hla/get_allele.cgi?B*57:96 |
| B57:97 | https://www.ebi.ac.uk/cgi-bin/ipd/imgt/hla/get_allele.cgi?B*57:97 |
| B57:98Q | hhttps://www.ebi.ac.uk/cgi-bin/ipd/imgt/hla/get_allele.cgi?B*57:98Q |
| B57:99 | https://www.ebi.ac.uk/cgi-bin/ipd/imgt/hla/get_allele.cgi?B*57:99 |
| B57:100 | https://www.ebi.ac.uk/cgi-bin/ipd/imgt/hla/get_allele.cgi?B*57:100 |
| B57:101 | https://www.ebi.ac.uk/cgi-bin/ipd/imgt/hla/get_allele.cgi?B*57:101 |
| B57:102 | https://www.ebi.ac.uk/cgi-bin/ipd/imgt/hla/get_allele.cgi?B*57:102 |
| B57:103 | https://www.ebi.ac.uk/cgi-bin/ipd/imgt/hla/get_allele.cgi?B*57:103 |
| B57:104 | https://www.ebi.ac.uk/cgi-bin/ipd/imgt/hla/get_allele.cgi?B*57:104 |
| B57:107 | https://www.ebi.ac.uk/cgi-bin/ipd/imgt/hla/get_allele.cgi?B*57:107 |
| B57:108 | https://www.ebi.ac.uk/cgi-bin/ipd/imgt/hla/get_allele.cgi?B*57:108 |
| B57:109 | https://www.ebi.ac.uk/cgi-bin/ipd/imgt/hla/get_allele.cgi?B*57:109 |
| B57:110 | https://www.ebi.ac.uk/cgi-bin/ipd/imgt/hla/get_allele.cgi?B*57:110 |
| B57:112 | https://www.ebi.ac.uk/cgi-bin/ipd/imgt/hla/get_allele.cgi?B*57:112 |
| B57:113 | https://www.ebi.ac.uk/cgi-bin/ipd/imgt/hla/get_allele.cgi?B*57:113 |
| B57:114 | https://www.ebi.ac.uk/cgi-bin/ipd/imgt/hla/get_allele.cgi?B*57:114 |
| B57:115 | https://www.ebi.ac.uk/cgi-bin/ipd/imgt/hla/get_allele.cgi?B*57:115 |
| B57:116 | https://www.ebi.ac.uk/cgi-bin/ipd/imgt/hla/get_allele.cgi?B*57:116 |
| B57:119 | https://www.ebi.ac.uk/cgi-bin/ipd/imgt/hla/get_allele.cgi?B*57:119 |
| B57:120 | https://www.ebi.ac.uk/cgi-bin/ipd/imgt/hla/get_allele.cgi?B*57:120 |
| B57:121 | https://www.ebi.ac.uk/cgi-bin/ipd/imgt/hla/get_allele.cgi?B*57:121 |
| B57:123 | https://www.ebi.ac.uk/cgi-bin/ipd/imgt/hla/get_allele.cgi?B*57:123 |
| B57:124 | https://www.ebi.ac.uk/cgi-bin/ipd/imgt/hla/get_allele.cgi?B*57:124 |
| B57:125 | https://www.ebi.ac.uk/cgi-bin/ipd/imgt/hla/get_allele.cgi?B*57:125 |
| B57:126 | https://www.ebi.ac.uk/cgi-bin/ipd/imgt/hla/get_allele.cgi?B*57:126 |
| B57:127 | https://www.ebi.ac.uk/cgi-bin/ipd/imgt/hla/get_allele.cgi?B*57:127 |
| B57:128 | https://www.ebi.ac.uk/cgi-bin/ipd/imgt/hla/get_allele.cgi?B*57:128 |
| B57:131 | https://www.ebi.ac.uk/cgi-bin/ipd/imgt/hla/get_allele.cgi?B*57:131 |
| B57:132 | https://www.ebi.ac.uk/cgi-bin/ipd/imgt/hla/get_allele.cgi?B*57:132 |
| B57:133 | https://www.ebi.ac.uk/cgi-bin/ipd/imgt/hla/get_allele.cgi?B*57:133 |
| B57:134 | https://www.ebi.ac.uk/cgi-bin/ipd/imgt/hla/get_allele.cgi?B*57:134 |
| B57:135 | https://www.ebi.ac.uk/cgi-bin/ipd/imgt/hla/get_allele.cgi?B*57:135 |
| B57:136 | https://www.ebi.ac.uk/cgi-bin/ipd/imgt/hla/get_allele.cgi?B*57:136 |
| B57:137 | https://www.ebi.ac.uk/cgi-bin/ipd/imgt/hla/get_allele.cgi?B*57:137 |
| B57:138 | https://www.ebi.ac.uk/cgi-bin/ipd/imgt/hla/get_allele.cgi?B*57:138 |
| B57:140 | https://www.ebi.ac.uk/cgi-bin/ipd/imgt/hla/get_allele.cgi?B*57:140 |
| B57:141 | https://www.ebi.ac.uk/cgi-bin/ipd/imgt/hla/get_allele.cgi?B*57:141 |

In other embodiments of the methods to produce an HLA fusion protein, or the isolated HLA fusion protein according to the invention, the variant HLA-B57 polypeptide is identical to the extracellular domain of any naturally occurring HLA-B57 heavy chain polypeptide which comprises a full length extracellular domain, truncated before the "connecting peptide" region, the region of the HLA-B57 heavy-chain following the extracellular domain, and preceding the transmembrane domain, in addition to being characterized by an E at position 46, and an R at position 97.

In particular embodiments of the methods to produce an HLA fusion protein, or the isolated HLA fusion protein according to the invention, the variant HLA-B57 polypeptide is identical to the extracellular domain of any naturally occurring HLA-B57 heavy chain polypeptide which comprises a full length extracellular domain, truncated before the least 3, 4, 5, or 6 amino acid residues of the "connecting peptide" region, in addition to being characterized by an E at position 46, and an R at position 97.

In more particular embodiments of the methods to produce an HLA fusion protein, or the isolated HLA fusion protein according to the invention, the variant HLA-B57 heavy-chain polypeptide is identical to the extracellular domain of any naturally occurring HLA-B57 heavy chain polypeptide which comprises a full length extracellular domain, but lacking the 5 C-terminal amino acid residues of the "connecting peptide" region, in addition to being characterized by an E at position 46, and an R at position 97.

In particular embodiments, the variant HLA-B57 polypeptide is identical to the first 280 amino acids encoding the extracellular portion of any naturally occurring HLA-B57 heavy chain polypeptide, counting from the GSH motif of the alpha 1 domain of the extracellular domain, apart from being characterized by an E at position 46, and an R at position 97.

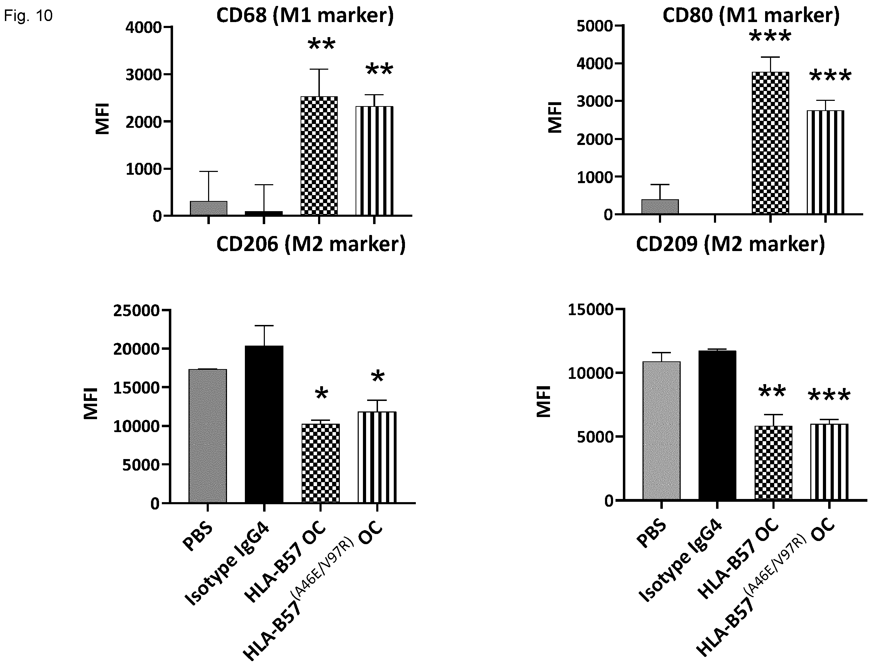

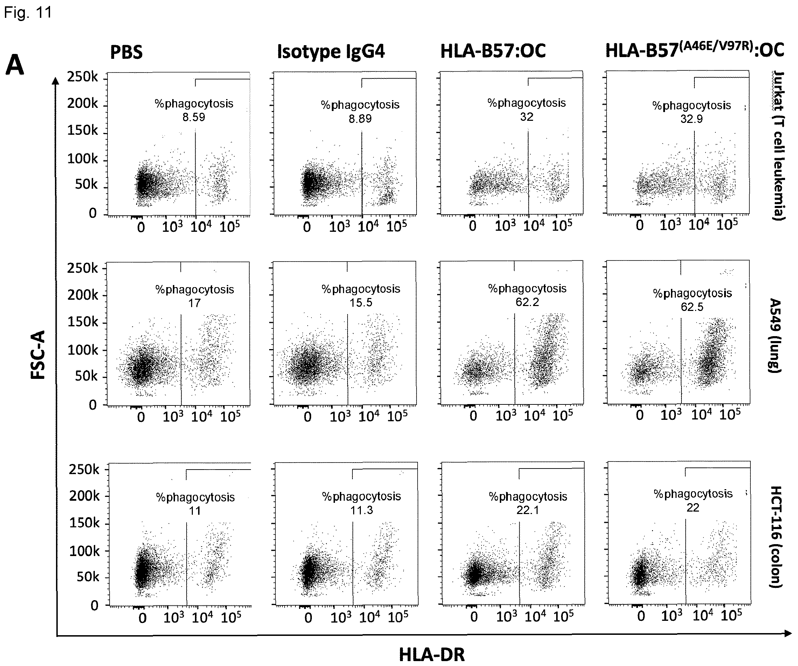

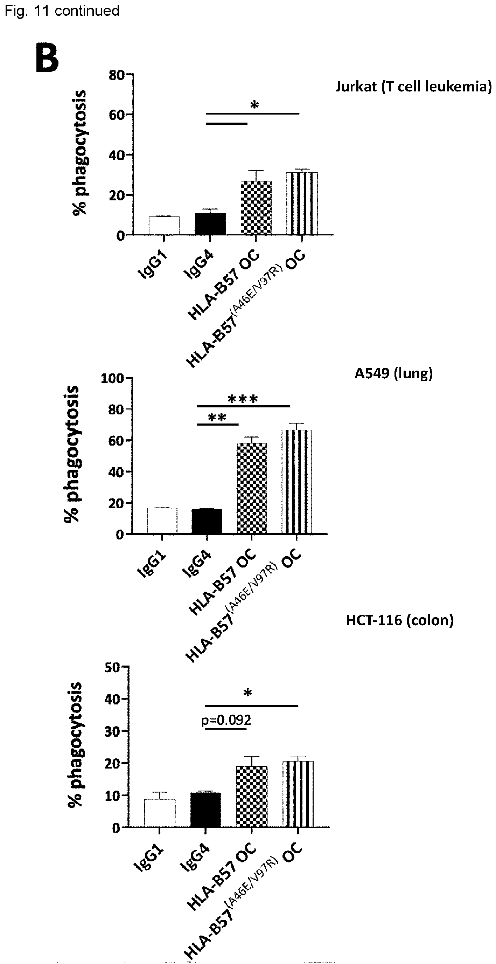

In alternative embodiments of the isolated HLA fusion protein according to the invention, the variant HLA-B57 polypeptide comprises a sequence identical to the extracellular domain of a naturally occurring HLA-B57 polypeptide, for example, SEQ ID NO 002, other than a single differing amino acid residue (wherein the single differing amino acid residue is not position 46, or 97). In other words, in addition to the one, or in some cases, two amino acid substitutions at positions 46, and/or 97, the variant HLA-B57 polypeptide according to this embodiment includes at least one further amino acid substitution compared to the naturally occurring HLA-B57 molecule from which it is derived. According to this embodiment, the resulting HLA fusion protein has a similar, or improved biological function to all previous embodiments of the isolated HLA fusion protein according to the invention. Specifically, the function of the HLA fusion protein can be characterized by means and methods demonstrated in the examples herein, namely in comparison to a similar construct lacking the amino acid substitutions at positions 46, and 97, the HLA fusion protein is characterized by inducing:
a. at least a 3-fold increase in the viability of the mammalian cell expressing said HLA fusion protein, for example in the method indicated in Fig 3A; and/or
b. at least a 10-fold increase in expression titer of said HLA fusion protein determined in cell culture supernatant as in Fig 3B;
   and
c. at least an 80%, particularly 90% upregulation in the level of CD68, CD206 and/or CD209 expression on the cell surface of macrophages (in an assay such as that demonstrated in Fig. 10); and/or
d. at least an 80%, particularly 90% of the level of phagocytosis of tumour cells by macrophages (as determined in Fig. 11); and/or
e. at least a 2-fold reduction, or particularly a 3-fold reduction in tumour volume when administered in vivo, as shown in Fig. 12.

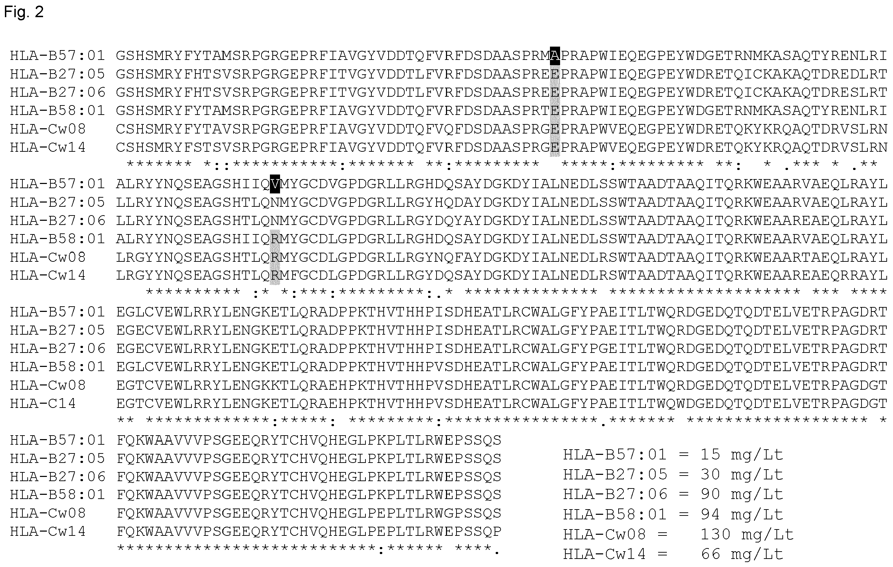

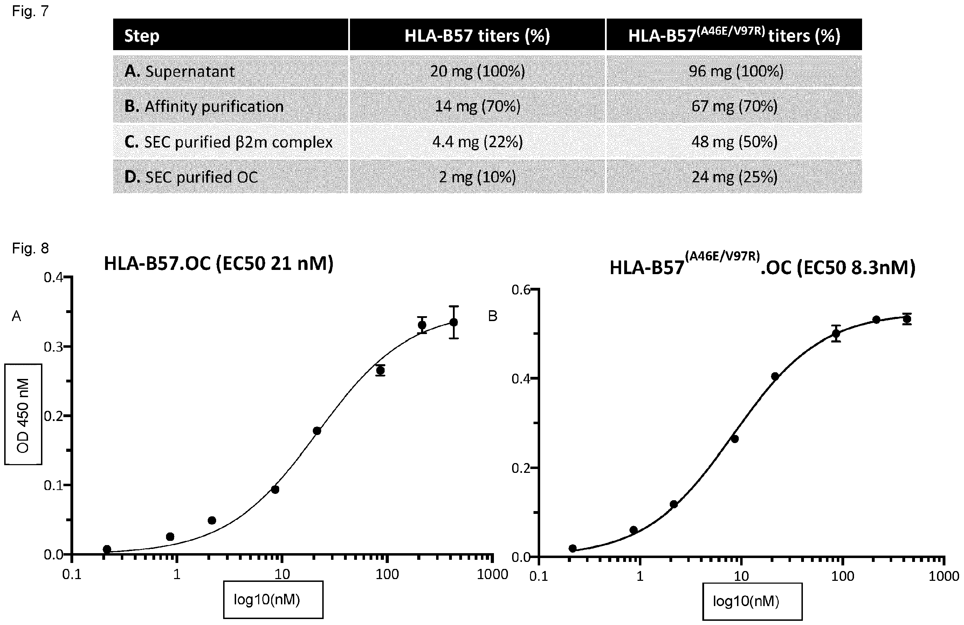

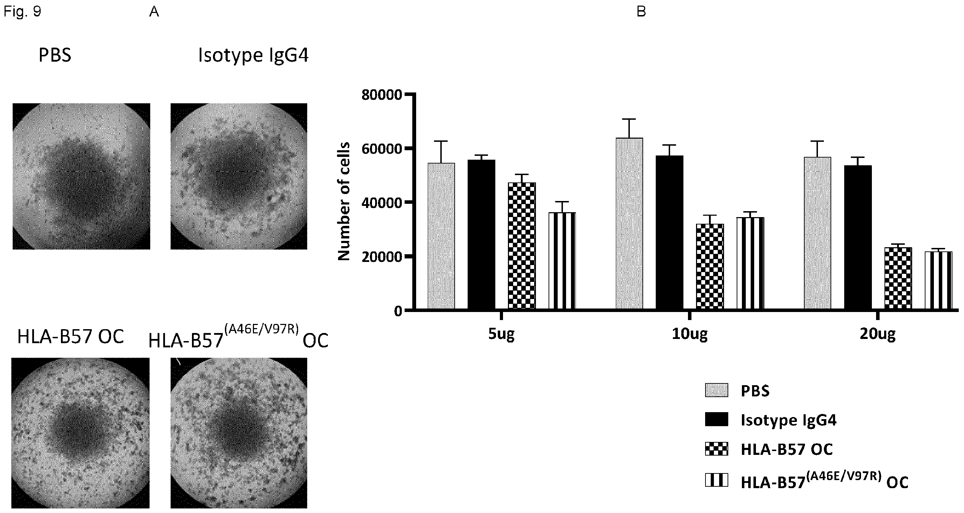

The data in Fig. 2 presented in the examples show that diverse HLA heavy chain structures bearing an A46E and an V97R correlate with higher yields of HLA heavy chain expression by cell lines when compared to the naturally occurring HLA-B57 polypeptide. Fig.3 to Fig. 7 demonstrate that changing two amino acids (A46E and V97R) in the extracellular portion of natural HLA-B57:01 (SEQ ID NO 001) heavy chain polypeptide greatly improves the yield, and reduces aggregation of a β2m in complex with an HLA Ig Fc fusion protein (HLA-B57^{(A46E, V97R)}. β2m), and also the HLA-B57^{(A46E, V97R)} open conformer lacking β2m (HLA-B57^{(A46E, V97R)}). The amino acid substitutions increase cell viability, production titer and decreases high and low molecular weight species, as indicated by the predominance of a single peak in HLPC analysis of HLA fusion proteins comprising the variant HLA-B57 polypeptide. Importantly, these amino acid substitutions do not compromise the binding to LILRB2 of an HLA open conformer (HLA-B57^{(A46E, V97R)} OC) (Fig. 8), nor the ability of HLA-B57(^{A46E, V97R})OC to increase the killing potential of CD8 T cells or polarization of M1 macrophages (Fig. 9 to 11). This confirms that position 46 and 97 do not compromise the immunomodulatory properties of the resulting HLA fusion protein. Additionally, the β2m dissociated HLA-B57^{(A46E, V97R)}.OC has a potent immunomodulatory effect and anti-cancer therapeutic efficacy as demonstrated in vivo using a colon cancer mouse model (Fig. 12).

In particular embodiments of the method for producing an HLA fusion protein, or the isolated HLA according to the invention, an open conformer derived from the variant HLA fusion protein has an improved binding to LILRB2 (UniProt Q8N423) compared to an equivalent compound comprising the naturally-occurring HLA heavy chain peptide from which it is derived. In other words, binding saturation of LILRB2 with an HLA-B57 (^{A46E, V97R)} fusion protein can be achieved at a lower concentration compared an equivalent construct comprising the naturally-occurring HLA heavy chain peptide from which it is derived. LILRB2 binding saturation according to the invention particularly refers to measurements using an enzyme-linked immunoassay, where HLA fusion protein is titrated onto immobilized biotinylated LILRB2, and detected with an anti-Ig secondary antibody. In other particular embodiments, said variant HLA heavy chain incorporated into an open conformer HLA fusion protein binds to LILRB2 receptor with an EC50 of equal to, or less than (≤) about 21nM, particularly ≤ about 15nM, more particularly ≤ about 10nM, indicating more than about a 2-fold increase in LILRB2 binding saturation.

### Stabilizing and linking peptides

The HLA fusion protein according to any aspect of the methods, or the isolated HLA fusion protein according to the invention specified above, comprises an additional polypeptide conferring stability during expression and purification to the HLA-B57 portion of the fusion protein. The presence of stabilizing portion of the HLA fusion protein increases the yield and solubility by reducing degradation and oligomerization of the HLA fusion protein, as well as increasing viability of the cell expressing the fusion protein.

In particular embodiments of the method to produce an HLA fusion protein, or the isolated HLA fusion protein, the stabilizing polypeptide of the HLA fusion protein is a human Ig Fc polypeptide. In particular embodiments, the stabilizing peptide of the HLA fusion protein is an isotype IgG Fc. An IgG Fc stabilizing peptide domain delivers an added advantage during purification of the HLA fusion protein, by enabling absorption to a protein A or G coated surface. The Fc portion may also prolong the in vivo half-life of a molecule *in vivo.* The inventors consider a possible alternative that may provide similar benefits if attached to the functional HLA polypeptides in lieu of Ig Fc could be bovine serum albumin. Previous work by the inventors has established that an albumin molecule, such as bovine serum albumin, may also serve as a stabilizing polypeptide. It is also known that PEGylation can enhance the half-life of proteins in circulation, and is thus another feasible stabilizing polypeptide.

In particular embodiments of the method to produce an HLA fusion protein, or the isolated HLA fusion protein according to the invention, the HLA fusion protein comprises an IgG4 polypeptide. In more particular embodiments, the HLA fusion protein comprises an altered IgG4 S228P.dk molecule with the sequence SEQ ID NO 004. This is characterized by a mutation in the hinge region of the IgG4, where Proline (P) is substituted for serine (S) at position 228 of the original IgG4 antibody, and dK indicates a deletion of the last amino acid Lysine (K) on the original IgG4 sequence. These changes give the IgG4 format stability and less heterogenicity. Both changes are well established and used commonly in diverse Fc constructs.

In certain embodiments, the isolated HLA fusion protein is a dimer which comprises a first monomer and a second monomer, and each monomer independently of the other monomer comprises an HLA heavy chain polypeptide fused to an Ig Fc portion, the latter of which may associate via disulfide bonds.

In certain embodiments of the method to produce an HLA fusion protein, the variant HLA-B57 polypeptide is joined with the IgG polypeptide as part of a single polypeptide chain by a peptide linker, a short sequence of amino acids 5, 10, 15, or 20, or even 50 residues in length. In particular embodiments, the peptide linker is a non-immunogenic sequence rich in serine and glycine residues. In more particular embodiments, the peptide linker has the sequence SEQ ID NO 003.

In alternative embodiments of the method to produce an HLA fusion protein, or the isolated HLA fusion protein according to the invention, the HLA fusion protein additionally comprises a peptide epitope fragment.

### Nucleic acids

Another aspect of the invention provides a nucleic acid encoding the isolated HLA fusion protein according to any one if the aspects or embodiments of the invention recited above, where the encoded variant HLA-B57 polypeptide portion of the HLA fusion protein is characterized by an E46, and an R97. In particular embodiments, the nucleic acid encoding the HLA fusion protein comprises the sequence SEQ ID NO 016. In particular embodiments the nucleic acid also encodes a secretory signal permitting secretion of the cell to enable efficient purification of the HLA fusion protein. In other particular embodiments, the nucleic acid encoding the isolated fusion protein comprises the sequence designated SEQ ID NO 006, further encoding a secretory signal. In more particular embodiments, it essentially consists of SEQ ID NO 006.

Further aspects of the invention include a nucleic acid expression vector comprising the nucleic acid as specified above, and a promoter sequence operable in a cell. In particular embodiments, the promotor is suitable for expression in a eukaryotic cell. In more particular embodiments, a promotor for a mammalian cell is used.

A further aspect of the invention is an isolated cell comprising the HLA fusion protein, or a nucleic acid encoding the HLA fusion protein according to any previous aspects of the invention. These aspects of the invention encompass DNA or RNA-based delivery systems for HLA fusion proteins, or transfer of cells expressing the HLA fusion protein.

### Pharmaceutical Compositions and Administration

The data presented in the examples shows that an HLA fusion protein according to the invention, where the A46, and V97 of the extracellular domains of the HLA-B*57:01 polypeptide have been replaced by an E46, and an R97 by targeted amino acid substitutions, binds to LILRB2, increases the killing capacity of CD8 T cells, and provokes anti-tumor immune responses *in vivo.* The amino acid substitutions in the variant HLA-B57 polypeptide provided by the invention are advantageous in that they increase the yield of the resulting HLA fusion protein comprising said variant HLA-B57 polypeptide, while maintaining the immunomodulatory effects of the molecule, including T cell killing, macrophage activation and phagocytosis, and in vivo therapeutic efficacy (Fig. 9 to 12).

One aspect of the invention relates to a pharmaceutical composition comprising at least one of the HLA fusion proteins, or the nucleic acid encoding the HLA fusion protein as specified herein, and at least one pharmaceutically acceptable carrier, diluent or excipient, for use in treating a malignant neoplastic disease. In some embodiments, the pharmaceutical composition comprises at least two pharmaceutically acceptable carriers, such as those described herein.

In particular embodiments, the malignant neoplastic disease in which the pharmaceutical HLA fusion protein formulation is used is a solid cancer. In more particular embodiments, the solid cancer is lung cancer, or metastatic lung cancer. In other particular embodiments, the cancer is a form colorectal cancer, or metastatic colon cancer.

In other particular embodiments, the pharmaceutical HLA fusion protein formulation is used to treat a blood-cell derived cancer. In more particular embodiments, it is used to treat T cell leukemia.

In certain embodiments of the invention, the HLA fusion protein of the present invention is typically formulated into pharmaceutical dosage forms to provide an easily controllable dosage of the drug and to give the patient an elegant and easily handled product.

The dosage regimen for the HLA fusion protein of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the HLA fusion protein of the invention may be administered in a single daily dose, or the total daily dosage may be administered in divided doses of two, three, or four times daily.

Many procedures and methods for preparing pharmaceutical compositions are known in the art, see for example L. Lachman et al. The Theory and Practice of Industrial Pharmacy, 4th Ed, 2013 (ISBN 8123922892).

### Combination medicaments

Another aspect of the invention is pharmaceutical composition comprising an HLA fusion protein according to the invention, or a nucleic acid encoding said HLA fusion protein, formulated for administration in combination with a checkpoint inhibitory agent. The inventors have previously found HLA-B57 IgG4 fusion protein compounds to enhance the effect of checkpoint inhibitors, and reason similar molecules based on the variant HLA-B57 heavy chain polypeptide will provide a similar effect when administered in combination, as the molecule shows similar immunomodulatory action in the in vitro assays tested in the examples.

Another aspect of the invention is a checkpoint inhibitor for use in treating a malignant neoplastic disease, formulated for administration in combination with an HLA fusion protein, or a nucleic acid, or a vector encoding the fusion protein according to the invention.

The checkpoint inhibitory agent according to the invention is capable of disrupting the inhibitory signalling cascade that limits immune cell activation, and in particular T cell activation. In certain embodiments, the checkpoint inhibitory agent is an antibody fragment, or an antibody-like molecule capable of binding to one of CTLA-4, PD-1, PD-L1, or PD-L2 with a dissociation constant of at least 10⁻⁷ M⁻¹.

Particularly, the checkpoint inhibitor agent is a non-agonist CTLA-4 ligand, a non-agonist PD-1 ligand, a non-agonist PD-L1 ligand, or a non-agonist PD-L2 ligand, which does not lead to attenuated T cell activity when binding to CTLA-4, PD-1, PD-L1 or PD-L2, respectively, on the surface on a T-cell. In certain embodiments, the term "non-agonist CTLA-4 ligand" or "non-agonist PD-1 ligand" covers both antagonists of CTLA-4 or PD-1 and ligands that are neutral vis-à-vis CTLA-4 or PD-1 signalling. In some embodiments, non-agonist CTLA-4 ligands used in the present invention are able, when bound to CTLA-4, to sterically block interaction of CTLA-4 with its binding partners CD80 and/or CD86 and the non-agonist PD-1 ligands used in the present invention are able, when bound to PD-1, to sterically block interaction of PD-1 with its binding partners PD-L1 and/or PD-L2.

In certain embodiments, the checkpoint inhibitory agent disrupts inhibitory signalling cascades via a capacity to bind to CTLA-4 with a dissociation constant of at least 10⁻⁷ M⁻¹, 10⁻⁸ M⁻¹ or 10^{- 9} M⁻¹ and which inhibits the biological activity of its respective target. A non-agonist PD-1 ligand or a non-agonist PD-L1 (PD-L2) ligand in the sense of the invention refers to a molecule that is capable of binding to PD-1 (PD-L1, PD-L2) with a dissociation constant of at least 10⁻⁷ M⁻¹, 10⁻⁸ M⁻¹ or 10⁻⁹ M⁻¹ and which inhibits the biological activity of its respective target. In particular embodiments, the checkpoint inhibitory agent is an antibody, more particularly an antibody specific for PD-1, which inhibits interactions with PD-L1 to enhance T cell responses.

In particular embodiments of the pharmaceutical composition for combination treatment, the checkpoint inhibitory agent is a checkpoint inhibitor antibody selected from the clinically available antibody drugs ipilimumab (Bristol-Myers Squibb; CAS No. 477202-00-9), tremelimumab (CAS 745013-59-6), nivolumab (Bristol-Myers Squibb; CAS No 946414-94-4), pembrolizumab (Merck Inc.; CAS No. 1374853-91-4), pidilizumab (CAS No. 1036730-42-3), atezolizumab (Roche AG; CAS No. 1380723-44-3), avelumab (Merck KGaA; CAS No. 1537032-82-8), durvalumab (Astra Zeneca, CAS No. 1428935-60-7), and cemiplimab (Sanofi Aventis; CAS No. 1801342-60-8).

An antibody fragment may be a Fab domain or a variable fragment (Fv) domain of an antibody, or a single-chain antibody fragment (a fusion protein consisting of the variable regions of light and heavy chains of an antibody connected by a peptide linker). The inhibitor may also be a single domain antibody, consisting of an isolated variable domain from a heavy or light chain. Additionally, an antibody may also be a heavy-chain antibody consisting of only heavy chains such as antibodies found in camelids. An antibody-like molecule may be a repeat protein, such as a designed ankyrin repeat protein (Molecular Partners, Zürich).

In certain embodiments, the combination therapy comprises two distinct dosage forms, for example, said HLA fusion protein is provided as a dosage form for intra-tumoral delivery, or local delivery in the vicinity of the tumour, for example, by subcutaneous injection, or intra-tumoral injection into a solid tumour, and said checkpoint inhibitor agent is provided as a dosage form for systemic delivery, particularly by intravenous injection. However, said checkpoint inhibitor agent and said HLA fusion protein may also be delivered in two similar dosage forms.

Administration in combination, encompasses both simultaneous administration of the checkpoint inhibitor agent and the HLA fusion protein together, or in separate formulations, or administration of one substance immediately prior to, for example, in the week prior to, or immediately subsequent to, for example, in the week subsequent to, administration of a second substance. In some embodiments, administration of the two agents in combination refers to administration of one agent prior to, particularly in the month prior to the second agent. In other embodiments, one second is administered subsequent to, particularly in the weeks, or month subsequent to, the first agent. In other particular embodiments, the checkpoint inhibitor and the HLA fusion protein are administered in overlapping administration regimes.

### Medical treatment, Dosage Forms, Method of Manufacture

The pharmaceutical compositions for use according to the invention is provided for use in treating malignant neoplastic disease, encompassing various forms of cancer.

In particular embodiments of the pharmaceutical composition comprising an HLA fusion protein, it is provided for use to treat a type of liquid, or blood cancer. The inventors consider the characteristic T cell-exhaustion, and accessibility of circulating blood cancer cells to the T cell, and macrophage activation induced by pharmaceutical compositions according to the invention mean this combination is likely to be efficacious. In particular such embodiments, the pharmaceutical composition is provided for use in a patient diagnosed with T cell leukemia, as modelled herein with Jurkat cells. In other particular embodiments, the pharmaceutical composition is provided for use in a patient diagnosed with multiple myeloma, as modelled by the THP-1 AML cell line.

In other particular embodiments, the pharmaceutical composition comprising an HLA fusion protein is provided for use in a patient diagnosed with a solid cancer, or a metastasis of a solid cancer. In some particular embodiments, the pharmaceutical composition is for use in a patient diagnosed with lung cancer. In particular embodiments, the lung cancer is a form of non-small cell lung cancer. In other particular embodiments, the lung cancer is a form of small cell lung cancer, or carcinoma. In other particular embodiments, the pharmaceutical composition comprising and HLA fusion protein, or the immune checkpoint inhibitor is for use in a patient diagnosed with colon cancer. In particular embodiments, metastatic colon cancer.

In some embodiments of aspects of the invention relating to administration of a pharmaceutical composition according to the invention in addition to a checkpoint inhibition agent, it is provided for use in a form of malignant disease, or cancer, in which checkpoint inhibition therapy is approved for monotherapy, or combination therapy. In particular embodiments, the combination treatment is for use in a patient with colon cancer, particularly metastatic colon cancer. On other particular embodiments, the cancer is melanoma. In further particular embodiments, the cancer is pancreatic cancer. In still further particular embodiments the cancer is breast cancer.

The invention further encompasses, as an additional alternative aspect, the use of an HLA fusion protein, or of a pharmaceutical composition comprising an HLA fusion protein as specified in detail above, for use in a method of manufacture of a medicament for the treatment, or prevention of recurrence, of a form of cancer. Another alternative aspect, is a method of treating a patient diagnosed with a form of malignant neoplastic disease by administering an effective amount of a pharmaceutical composition comprising an HLA fusion protein as specified in detail above.

Dosage forms may be for parenteral administration such as subcutaneous, intravenous, intrahepatic or intramuscular injection forms. Optionally, a pharmaceutically acceptable carrier and/or excipient may be present.

The invention further encompasses the following items:
A. A method for producing an HLA fusion protein obtained by introducing amino acid substitutions into a naturally occurring HLA-B57 heavy-chain, wherein the method comprises introducing into a cell, particularly a eukaryotic cell, more particularly a mammalian cell,
   a. an HLA nucleic acid sequence encoding a polypeptide comprising a variant of the extracellular domain of a naturally occurring HLA-B57 heavy-chain, wherein the variant HLA-B57 heavy-chain has an E at position 46 and an R at position 97, wherein the positions numbers are defined sequentially by assigning the GSH motif of the HLA-B57:01 heavy chain with SEQ ID NO 001 to positions 1, 2 and 3, respectively, and
   b. a β2m nucleic acid sequence encoding β2m protein,
      both under control of a promoter sequence operable in said cell, and wherein incubating the cell under conditions where the HLA and the ß2m encoding nucleic acid sequences are expressed provides an HLA heavy-chain / β2m complex.
B. A method for producing an HLA fusion protein obtained by introducing amino acid substitutions into a naturally occurring HLA-B57 heavy-chain, wherein the method comprises introducing into a cell, particularly a eukaryotic cell, more particularly a mammalian cell, nucleic acid sequences encoding:
   a. an HLA fusion protein comprising:
      i. a variant of the extracellular domain of a naturally occurring HLA-B57 heavy-chain, and wherein the variant HLA-B57 polypeptide is characterized by an E at position 46 and an R at position 97 (as specified in item A);
      ii. a stabilizing polypeptide, particularly a stabilizing peptide comprising an IgG Fc, more particularly a stabilizing polypeptide comprising an IgG4 Fc; and
   b. a β2m nucleic acid sequence;
      under control of a promoter sequence operable in said cell, and wherein incubating the cell under conditions where the HLA-B fusion protein and the ß2m-encoding nucleic acid sequences are expressed, provides an HLA heavy-chain / β2m complex.
C. A method for producing an HLA fusion protein comprising the following steps:
   a. an amino acid substitution step, introducing amino acid changes into a naturally occurring extracellular domain of an HLA-B57 heavy-chain to provide a variant HLA-B57 polypeptide characterized by an E at position 46 and/or an R at position 97, wherein the amino acid residues are numbered as specified in item A;
   b. in an expression step, introducing into a cell, particularly a eukaryotic cell, more particularly a mammalian cell, nucleic acid sequences encoding:
      i. an HLA fusion protein comprising a variant HLA-B57 polypeptide according to a. and an IgG Fc polypeptide, and
      ii. a β2m polypeptide,
         both under control of a promoter sequence operable in said cell, wherein incubating the cell under conditions where the HLA-B57and the ß2m-encoding nucleic acid sequences are expressed provides an HLA heavy-chain / β2m complex.
D. The method according to any one of the items A to C, wherein the naturally occurring HLA-B57 heavy-chain is characterized by an A at position 46, and/or a V or W at position 97.
E. The method according to any one of the items A to C, wherein the naturally occurring HLA-B57 heavy-chain is not already characterized by an E at position 46, and an R at position 97.
F. The method according to any one of the items A to E, wherein the naturally occurring HLA-B57 heavy-chain is characterized by an A at position 46.
G. The method according to any one of the items A to F, wherein the naturally occurring HLA-B57 heavy-chain is characterized by a V at position 97.
H. The method according to any one of the items A to G, wherein the naturally occurring HLA-B57 heavy-chain is characterized by an A at position 46, and a V or a W at position 97.
I. The method according to any one of the items A to H, wherein the naturally occurring HLA-B57 heavy-chain sequence is SEQ ID NO 001.
J. The method according to any one of the items A to I, wherein the variant HLA-B57 polypeptide comprises, or consists of, the sequence SEQ ID NO 002.
K. The method according to any one of the items A to J, wherein the HLA fusion protein comprises, or essentially consist of, from N' to C' terminus:
   a. a variant HLA-B57 polypeptide as specified in any one of the items A to J, and
   b. a peptide linker, particularly a peptide linker between 5 and 20 amino acids in length, more particularly a peptide linker with the sequence SEQ ID NO 003, and
   c. a stabilizing polypeptide, particularly a stabilizing polypeptide comprising an IgG Fc, more particularly a stabilizing polypeptide comprising an IgG4 Fc, even more particularly an IgG4 polypeptide with the sequence SEQ ID NO 004,
   d. and optionally, a secretory signal, particularly a secretory signal with the sequence SEQ ID NO 019.
L. The method according to any one of the items A to K, wherein the HLA fusion protein nucleic acid sequence and the β2m nucleic acid sequence are present on a single nucleic acid vector molecule.
M. The method according to any one of the items A to K, wherein the HLA fusion protein nucleic acid sequence and the β2m nucleic acid sequence are present on different nucleic acid vector molecules.
N. The method according to item M, wherein the ratio of the nucleic acid vector comprising the HLA-B57 fusion protein nucleic acid sequence with respect to the nucleic acid vector comprising the β2m nucleic acid sequence is around 1.
O. The method according to any one of the items 1 to N, further comprising:
   a. a purification step, wherein an HLA fusion protein / β2m complex is purified from the supernatant of the cell;
   b. a dissociation step, wherein the purified HLA fusion protein / β2m complex is dissociated under conditions wherein an isolated HLA fusion protein is separated from an isolated β2m polypeptide;
   c. and optionally, a desalting and refolding step, wherein the isolated HLA fusion protein polypeptide is brought to a physiological pH.
P. The method according to item O, wherein the dissociation step is carried out by treatment under acidic conditions, particularly at, or below, pH 3.
Q. The method according to any one of the items A to P, wherein the stabilizing polypeptide comprises an IgG Fc, and wherein in the purification step, the HLA fusion protein / β2m complex is captured by a surface linked to protein G.
R. The method according to any one of the items A to Q, wherein the HLA fusion protein is expressed in a Chinese hamster ovarian cell.
S. An isolated HLA fusion protein comprising:
   a. a variant HLA-B57 polypeptide, wherein said variant HLA-B57 polypeptide is a variant of the extracellular portion of a naturally occurring HLA-B57 heavy-chain, and wherein
      the variant HLA-B57 polypeptide is characterized by an E at position 46, and an R at position 97, wherein the positions numbers are defined sequentially by assigning the G, S, H motif of the HLA-B57:01 heavy-chain with the sequence SEQ ID NO 001 to the positions 1, 2 and 3, respectively; and
   b. a stabilizing polypeptide, particularly an Ig Fc, more particularly an IgG Fc, still more particularly an IgG4 Fc.
T. The isolated HLA fusion protein according to item S, comprising:
   a. the variant HLA-B57 polypeptide as specified according to item S, particularly a variant HLA-B57 polypeptide comprising, or essentially consisting of the sequence SEQ ID NO 002, and
   b. an IgG4 Fc comprising, or essentially consisting of SEQ ID NO 004,
      particularly wherein the isolated HLA fusion protein comprises, or essentially consists of, a sequence selected from SEQ ID NO 005, or SEQ ID NO 020 (comprising a secretory signal), or SEQ ID NO 015 (lacking a secretory signal post secretion), particularly wherein the HLA fusion protein comprises or essentially consists of a polypeptide with the sequence SEQ ID NO 015.
U. The isolated HLA fusion protein according to item S or T, comprising or consisting of an amino acid sequence identical to SEQ ID NO 015, or identical to SEQ ID NO 015 apart from a single differing amino acid residue, wherein in comparison an isolated fusion protein comprising the equivalent naturally occurring HLA-B57:01 sequence, the HLA fusion protein is characterized by:
   a. at least a 3-fold increase in the viability of the mammalian cell expressing said HLA fusion protein, and/or
   b. at least a 10-fold increase in titer of said HLA fusion protein determined in cell culture supernatant,
      and in an *in vitro* assay, a capacity to induce:
   c. at least an 80%, particularly 90% upregulation in the level of CD68, CD206 and/or CD209 expression on the cell surface of macrophages and/or
   d. at least an 80%, particularly 90% of the level of macrophage phagocytosis of tumour cells
   and/or in an *in vivo* mouse tumour model a capacity to induce:
   e. at least a 2-fold reduction, or particularly a 3-fold reduction in tumour volume.
V. A nucleic acid encoding the HLA fusion protein according to any one of the items S to U, particularly a nucleic acid comprising the sequence SEQ ID NO 016 (core HLA fusion protein structure only), particularly a nucleic acid comprising, or essentially consisting of, SEQ ID NO 006 (HLA fusion protein and secretion signal).
W. A vector comprising the nucleic acid of item V under control of a promoter sequence operable in a cell, particularly a eukaryotic cell, more particularly a mammalian cell.
X. A cell comprising the HLA fusion protein according to any one of the items S to U, or the nucleic acid according to item V, or the vector according to item W.
Y. An isolated HLA fusion protein according to any one of the items S to U or the nucleic acid according to item V, for use as a medicament, or for use in the manufacture of a medicament, particularly for use as a medicament to treat cancer.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Description of the Figures

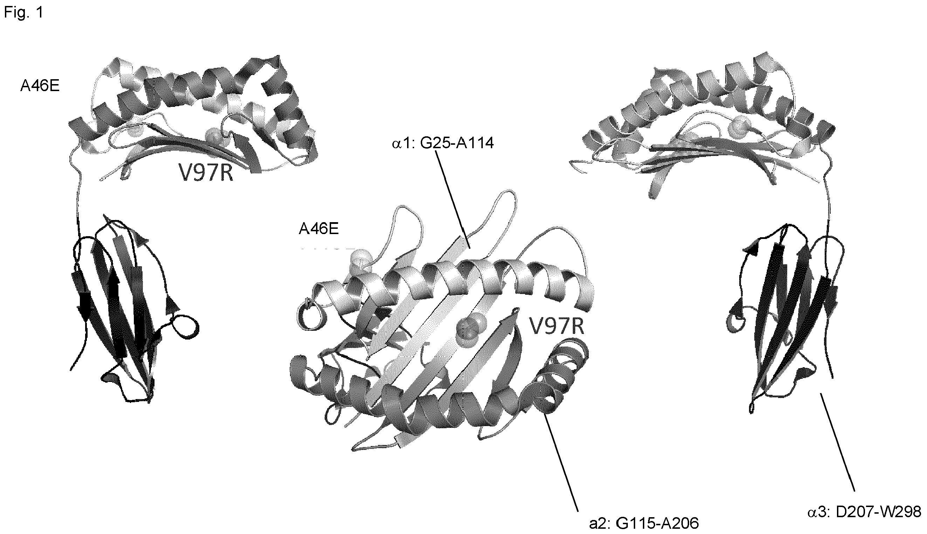

- Fig. 1: shows the topological structure of HLA-B57:01 without the β2m binding partner. The mutated residues A46E and V97R are highlighted as spheres. The three alpha domains (α1, α2 & α3) of the extracellular domain are color coded in the indicated different shades of grey.
- Fig. 2: shows glutamic acid (E) & Arginine (R) amino acids present in the extracellular domains of diverse HLA molecules (HLA-B57:01: SEQ ID NO 001; HLA-B27:05: SEQ ID NO 007; HLA-B27:06: SEQ ID NO 008; HLA-B58:01: SEQ ID NO 009; HLA-CW08: SEQ ID NO 010; HLA-CW14: SEQ ID NO 011) correlate with increase expression using transient transfection in CHO systems. Amino acid substitutions of A46E and V97R in HLA-B57 are highlighted.
- Fig. 3: shows selection of expression cell clones for HLA B57.β2m (DGC8-T39, DGC8-T64, & DGC8-73) and HLA-B57^{(A46E/V97R)}.β2m (DGC8-T54, DGC8-T75 & DGC8-91) on the basis of cell viability (A) and expressed protein titers (B). Results demonstrate that non-variant HLA-B57.β2m cell viability and titers are significantly lower than HLA-B57^{(A46E/V97R)}.β2m for all clones.

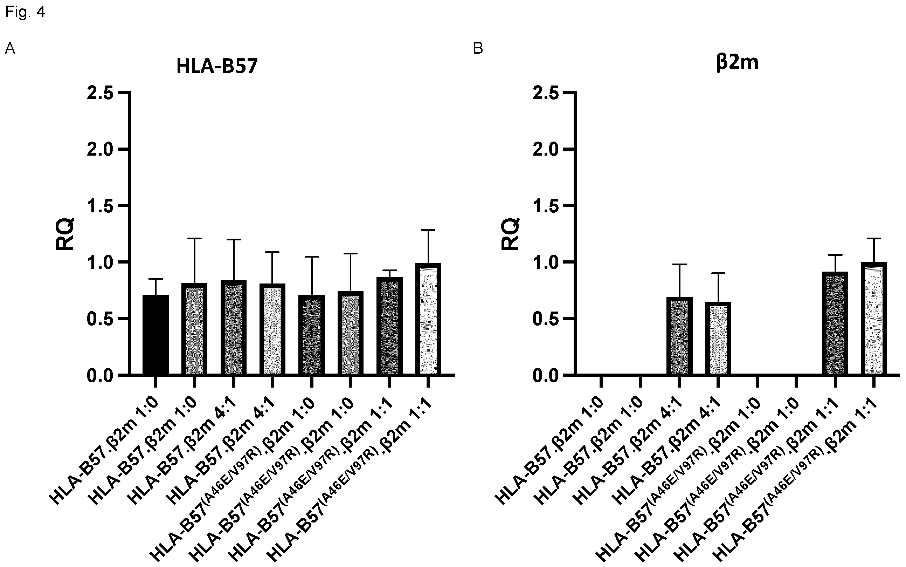

- Fig. 4: shows RNA profiles of (A) HLA-B57 and HLA-B57^{(A46E/V97R)}, and (B) β2m of clones co-expressing both molecules.

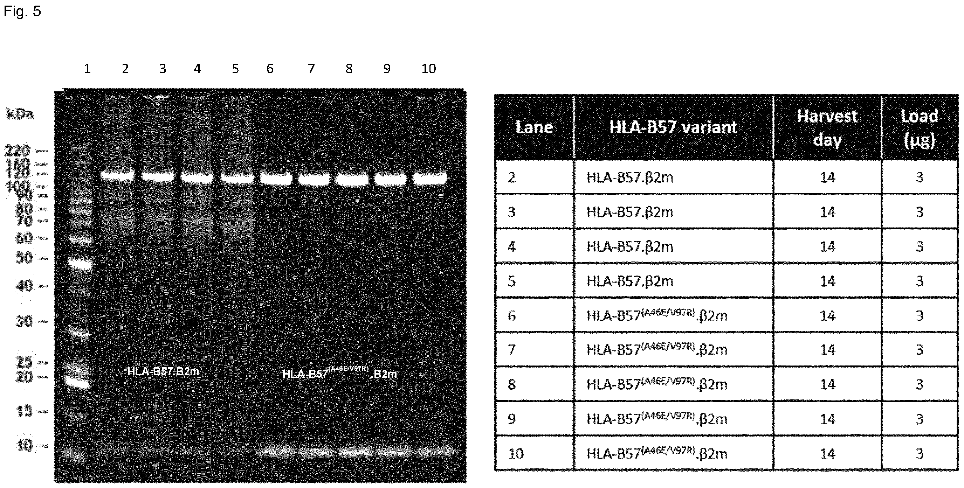

- Fig. 5: shows protein yield gels of purified HLA-B57.β2m (labeled iosHv1) HLA-B57^{(A46E/V97R)}.β2m (labeled iosHv2) and a summary of gel loading.

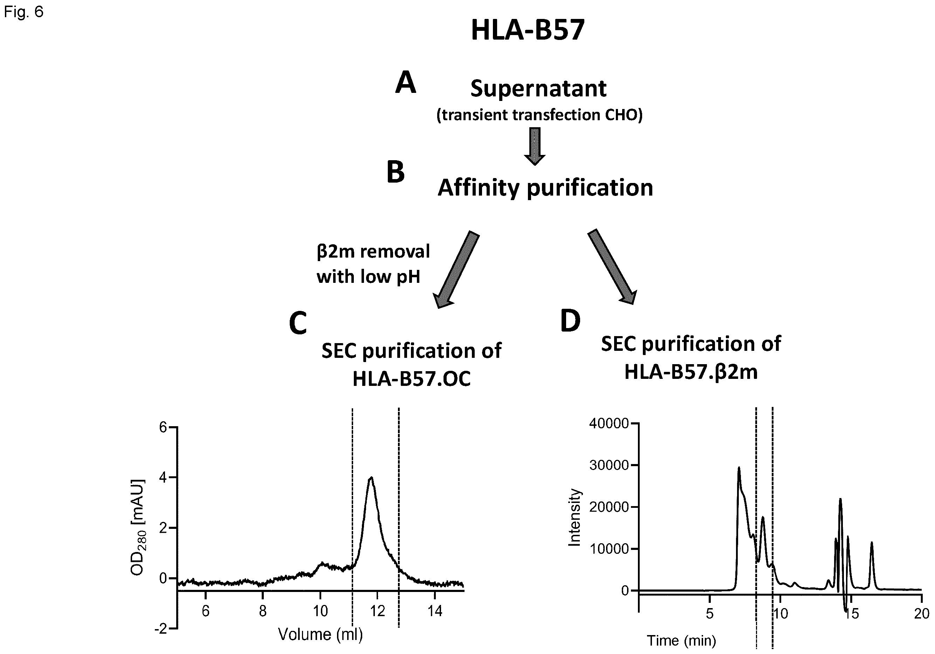

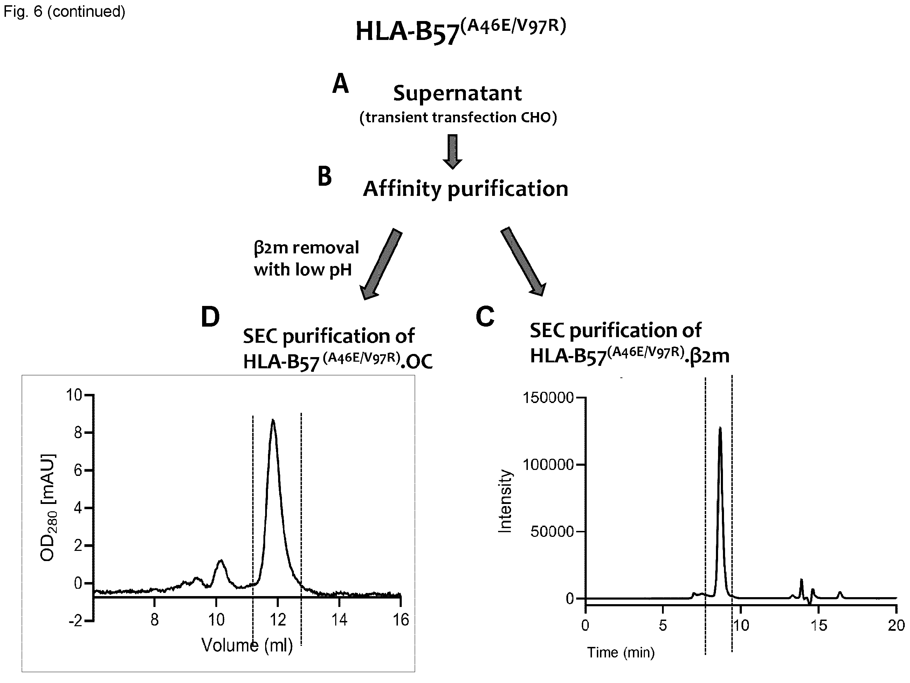

- Fig. 6: shows size exclusion chromatography (SEC) profiles of HLA-B57 and HLA-B57^{(A46E/V97R)} constructs purified in three steps. From CHO supernatant (A) affinity purification (B) two methods diverge. Open conformer purification is performed with an additional step for B2m removal, followed by SEC (C), and purification of B2m associated HLA-B57 is performed by direct SEC (D). Results demonstrate that HLA-B57 open conformers can be purified from both constructs. HLA-B57.β2m has high amount of high molecular weight (HMW) species and also low molecular weight species (LMW), with reduced monomer content whereas HLA-B57^{(A46E/V97R)}.β2m has significantly reduced HMW, LWM and high monomer content.
- Fig. 7: shows a table summarizing the associated yields of the indicated proteins from Fig. 6. at each step of the manufacturing process as in Fig. 6.
- Fig. 8: shows HLA-B57 amino acid substitutions increase the binding to LILRB2. Quantitative estimation of the binding affinities of LILRB2 with HLA-B57.OC, HLA-B57^{(A46E/V97R)}.OC by ELISA method. Where HLA-B57.OC has an EC50 of 21 nM, and HLA-B57^{(A46E/V97R)}.OC an EC50 of 8.3 nM.
- Fig. 9: shows HLA-B57.OC and HLA-B57^{(A46E/V97R)}.OC increase the killing potential of T cells. T cells were incubated with THP-1 AML cells in a cell-contact manner at a ratio of E:T (effector cells: T cells) 1:1, treated with compounds. (A) Representative cell pellets photographed on day 7 post plating. (B) Number of cancer cells obtained from co-cultures on day 5 post stimulation with titrated HLA-B57.OC or HLA-B57^{(A46E/V97R)}.OC and controls, measured using flow cytometry. *P < 0.05, **P < 0.01 and ****P < 0.0001 (one-way ANOVA with multiple-comparisons correction).
- Fig. 10: shows HLA-B57.OC and HLA-B57^{(A46E/V97R)}.OC induce the polarization of macrophages to an M1 phenotype associated with enhanced phagocytosis. Human primary monocytes were isolated and incubated with HLA-B57.OC or HLA-B57^{(A46E/V97R)}.OC compounds or controls and markers for M1 and M2 macrophages were evaluated after 6-7 days using flow cytometry. *P < 0.05, **P < 0.01, ***P < 0.001 and ****P < 0.0001 (one-way ANOVA with multiple-comparisons correction).
- Fig. 11: HLA-B57.OC & HLA-B57^{(A46E/V97R)}.OC induce the macrophage phagocytosis of cancer cells. Multiple cancer cell lines (Jurkat T cell leukemia, A549 lung cancer, and HCT-116 colon cancer) were incubated with macrophages which were pretreated for 6-7 days with compounds and phagocytosis was evaluated using flow cytometry after 16 hours of co-culture. (A) representative flow cytometry blots showing phagocytosis of cancer cells upon treatment with HLA-B57.OC or HLA-B57^{(A46E/V97R)}.OC in comparison to controls. (B) Percentage of phagocytosis. *P < 0.05, **P < 0.01, ***P < 0.001 and ****P < 0.0001 (one-way ANOVA with multiple-comparisons correction).
- Fig. 12: shows that monotherapy of open conformers of HLA-B57^{(A46E/V97R)}.OC reduce the size of tumors in the C38 murine syngeneic colon carcinoma model. Tumor volume mm³ of treated groups (n=8) as spider plots showing individual animals and response to (A) IgG4 or (B) HLA-B57^{(A46E/V97R)} OC treatment. Tumor volumes means were analyzed by two-way ANOVA followed by Bonferroni posthoc analysis, ****p<0.001.

### Examples

### Material and methods

### Generation of clone pools

CDNAs encoding HLA-B57-Fc (SEQ ID NO 012) & HLA-B57^{(A46E/V97R)}-Fc (SEQ ID NO 006) preceded by secretory leader signals were cloned separately into expression vectors (Probiogen). The vector constructs expressing HLA-B57-Fc & HLA-B57^{(A46E/V97R)}-Fc were co-transfected with a plasmid comprising a nucleic acid (SEQ ID NO 013) encoding the β2m protein (SEQ ID NO 014) by microporation (MP) using the NEON Transfection Kit (Life Technologies #MPK10096). CHO-DG44 starter cells were transfected at different ratios of HLA to β2m plasmid (4:1, 2:1, 1:1, 1:2). Selected clone pools were grown in standardized shaker flasks and with a defined cell seeding density of 4E5 vc/mL in 125 mL of PBG-CD-C4 supplemented medium including puromycin and methotrexate. Following adjusted selection pression with antibiotics, individual clone pools were selected for analysis. Measurement of viabilities and viable cell densities were performed using the Vi-CELL XR System, and Trypan blue cell exclusion method. Titer quantifications where measured at different time points (days) using an Octect RED machine (ForteBio, a Pall Division) with Protein A biosensors.

*Purification of HLA-B57.*β2*m and HLA-B57*^{*(A46E*/}*^{V97R)}.*β2*m and* β2*m removal procedure* Filtered supernatants containing the secreted HLA-B57.β2m and HLA-B57^{(A46E/V97R)}.β2m were used for affinity column purification. Purification of proteins and removal of β2m under acidic conditions was performed as a two-step purification protocol. As a first step, Protein G Sepharose [(4 Fast Flow) Sigma, #GE-17-0618-01)] beads were used to capture HLA-B57 + β2m and HLA-B57^{(A46E/V97R)} + β2m from supernatants. After an overnight incubation at 4 degrees on a rocker, the recovered beads were washed in PBS, and subsequently proteins were eluted using standard IgG-Elution Buffer (pH 2.8) (Pierce^{™} IgG Elution Buffer, Thermo Fischer #21004). A second step of size exclusion chromatography-based purification was performed to separate HLA-B57 & HLA-B57^{(A46E/V97R)} from β2m under acidic conditions. A Superdex 10/300 gel filtration column, pre-equilibrated in Sodium Citrate (100 mM, pH 3.0) was used for the separation. An injection of 0.5 ml of the protein at 2.0 mg/ml concentration was applied, and the desired HLA-B57.OC (SEQ ID NO 018) & HLA-B57^{(A46E/V97R)}.OC (SEQ ID NO 015) protein peak eluted at 12.7 ml and the peak for β2m eluted at 22.0 ml. These results demonstrate that the separation of β2m and purification of open conformer HLA-B57.OC & HLA-B57^{(A46E/V97R)}.OC is feasible under acidic conditions.

### Quantification of the interaction of LILRB2 HLA-B57.OC, and HLA-B57^{(A46E/}^{V97R)}.OC by ELISA

The quantification of the affinity of interaction of LILRB2 with HLA-B57.OC, HLA-B57^{(A46E/V97R)}.OC was conducted using the enzyme-linked immunosorbent assay (ELISA) method. Flat bottom Pierce^{™} Streptavidin coated high binding capacity 96 well plates (Pierce #15500) were used and 50 µl of c-terminally biotinylated antigen molecules (LILRB2, BPS Bioscience #100335) was immobilized at a final concentration of 5 µg/ml in PBS buffer. PBS and IgG isotype were used as negative controls. A serial dilution of HLA-B57.OC, or HLA-B57^{(A46E/V97R)}.OC (eight concentration points: 10, 2.5, 1, 0.25, 0.1, 0.025, 0.01, 0.0025 µg/ml) was applied (50 µl) in duplicate. An APC conjugated goat anti-human IgG antibody (Jackson Immuno Research #109-135-098) with 1:100 dilution in TBS (50 µl) was used for detection. Finally, 50 µl TBS in each well was added and a fluorescence scan was performed with APC excitation and emission wavelengths of 650 nm & 660 nm, respectively. Using Graphpad Prism v9.1.2, a three-parameter based log (agonist) vs. response model was used to determine the EC50 of the interaction.

### Antibody staining

Flow cytometry was performed on an LSR Fortessa Analyzer (BD Biosciences). T cell surface markers CD3, CD4 and CD8 were assessed by antibody staining (BioLegend) at a dilution of 1:100. HLA-DR expression on macrophages was assessed by antibody staining (BioLegend) at a dilution of 1:100. The macrophage polarization panel was performed using CD80, CD86, CD68, CD163, CD206 and CD209 antibodies (Biolegend) at a dilution of 1:100. All stains were performed on ice for 20 min, then were washed and resuspended according to standard practice.

### T cell killing assay

For the co-culture assay, human T cells were isolated from peripheral blood mononuclear cells (PBMCs) from healthy donors, stimulated with CD3/CD28-activator (ThermoFisher #11131D) and cultured in the presence of 50 U/ml rhlL-2 for 48 hours. T cells were then washed from the CD3/CD28-activator and subsequently mixed with the indicated human leukemia cells in a U-bottom 96-well plate in duplicate wells. Compounds were added at indicated concentrations to each well. Leukemia cells were stained prior to co-culture with CellTrace^{™} violet cell proliferation marker (ThermoFisher #C34557) according to manufacturer's instruction. The number of the plated cells, the E:T ratio and the duration of the co-cultures was tested for different leukemia cell lines and is indicated in the associated figure. Co-cultures were photographed using an inverted microscope, and T cells were stained with CD3, CD4 and CD8 antibodies and analyzed by LSR Fortessa Analyzer. Live cancer cells were positive for violet cell proliferation marker and negative for sytox red dead cell stain (ThermoFisher #S34859). Absolute count of both T cells and violet stain-positive cancer cells was measured using Bright count beads (ThermoFisher #C36590).

### Macrophage phagocytosis Flow-cytometry-based assay

Primary human donor-derived monocytes were isolated from PBMCs from a healthy donor and differentiated into macrophages by 5-7 days of culture in ImmunoCult medium (StemCell Technologies #10961) + 50ug/ml rhMCSF (StemCell #78057.1). On day 1 post plating compounds were added to wells at a concentration of 20µg/ml. On day 5-7 post plating, compounds were once again added to the macrophages and two downstream experiments were performed: 1) polarization of macrophages: For polarization studies cultured macrophages were analyzed by flowcytometry for expression of CD80, CD86, CD68, CD163, CD206 and CD209 (Biolegend) one day after the second treatment with compounds. 2) Phagocytosis: Target cells were plated on macrophages at a ratio of 0.5×10⁶ to 1×10⁶ macrophages in 12 well plates and analysed for phagocytosis 16 hours post plating. Target cells were stained with CellTrace^{™} violet cell proliferation marker prior to co-culture and thus could be differentiated from macrophages which were stained with HLA-DR by flow cytometry. Phagocytosis was evaluated as the percentage of violet stain-positive target cells from HLA-DR positive macrophages, as analysed using FlowJo v.10.6.1 (Tree Star). Each phagocytosis reaction was performed in technical duplicates. All biological replicates indicate independent human macrophage donors.

### In vivo treatments

C38 tumour fragments were injected subcutaneously into the right flanks of syngeneic female C57BL/6 mice. Once the tumour reached ± 50 mm³ in colon animals were distributed according to their individual tumour volume size and divided into groups displaying no statistical differences between them. Tumour diameters were measured using a calliper, and volume was calculated according to the formula, D/2×*d*² where *D* and *d* are the longest and shortest diameter of the tumour in mm, respectively. The Experimental design of injection time points of cells and injection of substances was established as follows: isotype IgG4 (10mg/Kg) biwk x 3; HLA-B57^{(A46E/V97R)}.OC (10 mg/Kg) biwk x 3; Biwk= bi-weekly

### Example 1: Correlation of HLA sequences with expression level

To optimize the expression profile of HLA-heavy chain open conformer Ig Fc fusion proteins for medical use, the protein sequences of a range of HLA heavy chain sequences were aligned to identify residues potentially associated with increased yield in a transient transfection system using Chinese hamster ovary cells (CHO). This comparison showed that specific glutamic acid (E) and arginine (R) amino acid residues present in diverse HLA molecules correlated with increase expression as measured by the concentration of recombinant protein measured in supernatant, suggesting these amino acid substitution changes may confer a variant HLA-B57^{(A46E/V97R)} with the superior expression levels of related HLA proteins when expressed in mammalian cells (Fig. 1 and 2).

The HLA-B57 haplotype is characterized by genetic linkage to immune phenotypes, and binds to innate receptors including the immunoglobulin-like receptor subfamily B member 2 (LILRB2), making the HLA-B57 protein sequence a particularly desirable HLA-heavy chain component for use in an HLA fusion protein. Substitution mutations were introduced into the amino acid sequence of an HLA-IgG4 Fc fusion protein based on the HLA-B57 alpha 1, 2 and 3 domains of the naturally occurring HLA-B57:01 protein (SEQ ID NO 001), exchanging an A at position 46 with an E, and a V at position 97 with an R (Fig. 1). DNA encoding the two HLA fusion proteins was then synthesized, comprising an IgG4 polypeptide linked by a short amino acid bridging sequence to one of two different HLA heavy chain polypeptide options; either the wildtype HLA-B57 extracellular polypeptide (SEQ ID NO 012, HLA-B57-Fc), or the mutant HLA-B57^{(A46E/V97R)} polypeptide (SEQ ID NO 006, HLA-B57^{(A46E/V97R)}-Fc). Each construct was cloned separately into expression vectors. The vector constructs expressing HLA-B57-Fc & HLA-B57^{(A46E/V97R)}-Fc were co-transfected into CHO cells in combination with a range of ratios of a second plasmid encoding the human β2m protein.

### Example 2: Improved expression of HLA-B57^{(A46E/}^{V97R)}.β2m

To test whether substitute amino acid residues affected the expression, or yield of recombinant HLA heavy chain IgG4 fusion proteins, CHO cell clones expressing the HLA-B57^{(A46E/V97R)}-Fc and HLA-B57-Fc construct were isolated and sub cloned, and the concentration of the resulting complexes comprising β2m together with either the wildtype (HLA-B57.β2m) or mutant HLA-B57 (HLA-B57^{(A46E/V97R) )}.β2m) fusion proteins was assessed by Protein A biosensors. HLA-B57^{(A46E/V97R) )}.β2m-producing clones showed increased cell and produced a significantly higher titer of HLA fusion protein in supernatant compared to HLA-B57:β2m-expressing control cells (Fig. 3). A structure comprising an alternative secretory signal in addition to the two mutations (SEQ ID NO 020), produced a similar supernatant yield of recombinant fusion protein. RNA levels of HLA-B57.β2m, HLA-B57^{(A46E/V97R)}.β2m and β2m were equal between cell clones, demonstrating that high titer expression of HLA-B57^{(A46E/V97R)}.β2m is not correlated with higher number of DNA copies, but rather associated to an intrinsic efficient folding, solubility, and stability of the new construct (Fig. 4). HPLC analysis of purified proteins showed that the supernatant of clones expressing the mutant HLA-B57^{(A46E/V97R)} construct yielded a higher ratio of low-molecular weight HLA heavy chain / β2m dimers, compared to increased high-molecular weight oligomers in the supernatant of the parent HLA-B57 construct (Fig. 5). The increased yield of the HLA-B57^{(A46E/V97R)} construct was also evident after purification and refolding of monomers comprising HLA open conformers without association with the β2m light chain (Fig. 6, summarized in Fig. 7).

### Example 3: Immunomodulatory characteristics of HLA-B57mutant

The activity of HLA-B57^{(A46E/V97R)} was compared to the HLA-B57^{(A46/V97)} parent structure in several assays to confirm the biological activity of HLA domain of an HLA IgG4 Fc fusion protein was not compromised by the addition of the two amino acid changes. ELISA analysis of open conformer compounds based on the non-variant or variant HLA-B57 heavy chain sequence demonstrated that the two amino acid substitutions did not reduce binding to LILRB2, and indeed the variant HLA fusion protein showed a improved, lower EC50 value of LILRB2 binding saturation (Fig. 8). The immunomodulatory effect of the HLA-B57^{(A46/V97)} open conformers was comparable to controls lacking the amino acid substitutions in assays measuring the capacity to increase the killing potential of T cells (Fig. 9), and the polarization (measured by representative surface phenotype markers) and phagocytosis capacity of M1 macrophages cultured with a range of tumor cell lines (Fig. 10 and 11). Lastly, the open conformer HLA-B57^{(A46E/V97R)}-Fc open conformer molecule demonstrated therapeutic immunostimulatory action *in vivo,* inhibiting a mouse model of colon cancer growth when delivered intraperitonially (Fig. 12).

### Sequences:

SEQ ID NO 002 variant HLA-B57 heavy chain extracellular domain polypeptide 
GSHSMRYFYTAMSRPGRGEPRFIAVGYVDDTQFVRFDSDAASPRMEPRAPWIEQEGPEYWDGETRNMK
ASAQTYRENLRIALRYYNQSEAGSHIIQRMYGCDVGPDGRLLRGHDQSAYDGKDYIALNEDLSSWTAA
DTAAQITQRKWEAARVAEQLRAYLEGLCVEWLRRYLENGKETLQRADPPKTHVTHHPISDHEATLRCW
 
ALGFYPAEITLTWQRDGEDQTQDTELVETRPAGDRTFQKWAAVVVPSGEEQRYTCHVQHEGLPKPLTL

RWEPSSQS
SEQ ID NO 015 variant HLA-B57 IgG4 Fc fusion protein

GSHSMRYFYTAMSRPGRGEPRFIAVGYVDDTQFVRFDSDAASPRMEPRAPWIEQEGPEYWDGETRNMK
ASAQTYRENLRIALRYYNQSEAGSHIIQRMYGCDVGPDGRLLRGHDQSAYDGKDYIALNEDLSSWTAA
DTAAQITQRKWEAARVAEQLRAYLEGLCVEWLRRYLENGKETLQRADPPKTHVTHHPISDHEATLRCW
ALGFYPAEITLTWQRDGEDQTQDTELVETRPAGDRTFQKWAAVVVPSGEEQRYTCHVQHEGLPKPLTL
RWEPSSQSGGGGSGGGGSESKYGPPCPPCPAPEFLGGPSVFLFPPKPKDTLMISRTPEVTCVVVDVSQ
EDPEVQFNWYVDGVEVHNAKTKPREEQFNSTYRVVSVLTVLHQDWLNGKEYKCKVSNKGLPSSIEKTI
SKAKGQPREPQVYTLPPSQEEMTKNQVSLTCLVKGFYPSDIAVEWESNGQPENNYKTTPPVLDSDGSF
FLYSRLTVDKSRWQEGNVFSCSVMHEALHNHYTQKSLSLSLG

## Claims

1. A method for producing a human leukocyte antigen (HLA) fusion protein obtained by introducing one, or two amino acid substitutions into a naturally occurring HLA-B57 extracellular domain polypeptide, wherein the method comprises introducing into a cell, particularly a eukaryotic cell, more particularly a mammalian cell,
a. a nucleic acid sequence encoding an HLA fusion protein, said HLA fusion protein comprising:
i. a variant HLA-B57 polypeptide, wherein the variant HLA-B57 polypeptide is an HLA-B57 extracellular domain polypeptide **characterized by** glutamate (E) at position 46 and an arginine (R) at position 97; and
ii. an immunoglobulin (Ig) fragment crystallizable region (Fc) polypeptide, particularly an isotype G Ig (IgG) Fc, more particularly an isotype 4 IgG (lgG4) Fc; and
b. a nucleic acid sequence encoding a β2-microglobulin (β2m) protein;
each under control of a promoter sequence operable in said cell,
then culturing the cell under conditions where the HLA fusion protein encoding nucleic acid sequence and the β2m protein encoding nucleic acid sequence are expressed, to provide an HLA fusion protein / β2m protein complex.

2. A method for producing an HLA fusion protein comprising the following steps:
a. in an amino acid substitution step, replacing in a naturally occurring HLA-B57 extracellular domain polypeptide, the amino acid at position 46 with an E, and/or replacing the amino acid at position 97 with an R, to provide a variant HLA-B57 polypeptide; and
b. in an expression step, introducing into a cell, particularly a eukaryotic cell, more particularly a mammalian cell, nucleic acid sequences encoding:
i. an HLA fusion protein comprising said variant HLA-B57 polypeptide and an IgG Fc polypeptide, and
ii. a β2m protein,
both under control of a promoter sequence operable in said cell, to provide an HLA fusion protein / β2m protein complex.

3. The method according to claim 1 or 2, wherein the naturally occurring HLA-B57 extracellular domain polypeptide is **characterized by**:
i. an A at position 46, and
ii. a V at position 97;
particularly wherein the variant HLA-B57 polypeptide comprises, or essentially consists of, the sequence SEQ ID NO 002.

4. The method according to any one of the claims 1 to 3, wherein the HLA fusion protein comprises:
a. a variant HLA-B57 polypeptide as specified in any one of the claims 1 to 3,
b. an IgG Fc polypeptide, particularly an IgG4 F polypeptide c, more particularly an IgG4 Fc polypeptide with the sequence SEQ ID NO 004,
c. a peptide linker connecting the variant HLA-B57 polypeptide to the IgG Fc polypeptide, particularly a peptide linker between 5 and 20 amino acids in length, more particularly a peptide linker with the sequence SEQ ID NO 003,
and wherein optionally, the HLA fusion protein further comprises:
d. a secretory signal, particularly wherein the secretory signal is 16 to 30 amino acids in length, more particularly wherein the secretory signal is removed by cleavage during the process of secretion from the cell, still more particularly a secretory signal with the sequence SEQ ID NO 019.

5. An isolated HLA fusion protein comprising:
a. a variant HLA-B57 polypeptide,
- wherein the variant HLA-B57 polypeptide is a variant of a naturally occurring HLA-B57 extracellular domain polypeptide;
- and wherein the variant HLA-B57 polypeptide is **characterized by** an E at position 46, and an R at position 97; and
b. an Ig Fc polypeptide, more particularly an IgG Fc polypeptide, even more particularly an IgG4 Fc polypeptide.

6. The isolated HLA fusion protein according to claim 5,
- wherein the variant HLA-B57 polypeptide comprises, or essentially consists of, the sequence SEQ ID NO 002; and/or
- wherein the Ig Fc polypeptide comprises, or essentially consists of, the sequence SEQ ID NO 004,
- and wherein optionally, the variant HLA-B57 polypeptide and the Ig Fc polypeptide are joined by a peptide linker, particularly wherein the peptide linker is between 5 and 20 amino acids in length, more particularly wherein the peptide linker comprises, or essentially consist of, the sequence SEQ ID NO 003.

7. The isolated HLA fusion protein according to claim 5 or 6, wherein the isolated HLA fusion protein comprises, or essentially consists of, the sequence designated SEQ ID NO 015.

8. The isolated HLA for use according to any one of the claims 5 to 7, wherein the isolated HLA is in the form of a dimer comprising a first monomer and a second monomer;
- and wherein said first monomer comprises, or essentially consists of a first HLA fusion protein obtained by means of method according to any one of the claims 1 to 4, or an isolated HLA fusion protein according to in any one of the claims 5 to 7;
- and wherein said second monomer comprises, or essentially consists of a first HLA fusion protein obtained by means of method according to any one of the claims 1 to 4, or an isolated HLA fusion protein according to any one of the claims 5 to 7;
- particularly wherein said first monomer and second monomer are identical.

9. The method for producing an HLA fusion protein according to any one of the claims 1 to 4, or the isolated HLA fusion protein according to any one of the claims 5 to 8, wherein said HLA fusion protein has improved binding to LILRB2 compared to an equivalent HLA fusion protein comprising said the naturally occurring HLA-B57 extracellular domain polypeptide.

10. An isolated nucleic acid encoding the isolated HLA fusion protein according to any one of the claims 5 to 9, particularly an isolated nucleic acid comprising the sequence SEQ ID NO 016, particularly an isolated nucleic acid comprising, or essentially consisting of the sequence SEQ ID NO 006.

11. A nucleic acid expression vector comprising the nucleic acid according to claim 10, under control of a promoter sequence operable in a cell, particularly a eukaryotic cell, more particularly a mammalian cell.

12. A cell comprising the HLA fusion protein according to any one of the claims 5 to 9, or the isolated nucleic acid according to claim 10, or the vector according to claim 11.

13. A pharmaceutical composition for use in the treatment of a malignant neoplastic disease, comprising an HLA fusion protein obtained from a method as specified in any one of the claims 1 to 4, the isolated HLA fusion protein according to any one of the claims 5 to 9, the nucleic acid according to claim 10, or the nucleic acid expression vector according to claim 11.

14. A pharmaceutical composition for use according to claim 13,
- wherein the pharmaceutical composition is administered prior to, in combination with, or subsequent to a checkpoint inhibitory agent,
- particularly wherein the checkpoint inhibitory agent is selected from an antibody, an antibody fragment, or an antibody-like molecule,
- and particularly wherein said checkpoint inhibitory antibody is capable of binding to one of CTLA-4, PD-1, PD-L1, or PD-L2 with a dissociation constant of at least 10⁻⁷ M^{-1,},
- and particularly wherein said checkpoint inhibitory antibody is provided in a dosage form for systemic delivery.

15. A checkpoint inhibitory agent for use in the treatment of a malignant neoplastic disease, wherein the checkpoint inhibitor is administered in combination with an HLA fusion protein obtained from a method according to any one of the claims 1 to 4, or 9, the isolated HLA fusion protein according to any one of the claims 5 to 9, the nucleic acid according to claim 10, or the nucleic acid expression vector according to claim 11,
- particularly wherein the checkpoint inhibitory agent is selected from an antibody, an antibody fragment, or an antibody-like molecule,
- and particularly wherein said checkpoint inhibitory antibody is capable of binding to one of CTLA-4, PD-1, PD-L1, or PD-L2 with a dissociation constant of at least 10⁻⁷ M^{-1,},
- and particularly wherein said checkpoint inhibitory antibody is provided in a dosage form for systemic delivery.
